(19) Europäisches Patentamt
European Patent Office
Office européen des brevets

(11) EP 1 097 128 B1

(12) # EUROPÄISCHE PATENTSCHRIFT

(45) Veröffentlichungstag und Bekanntmachung des Hinweises auf die Patenterteilung:
**12.05.2004 Patentblatt 2004/20**

(21) Anmeldenummer: **99936489.6**

(22) Anmeldetag: **07.07.1999**

(51) Int Cl.[7]: **C07C 261/04**, C07C 257/10, C07C 257/22, A01N 37/52, A01N 43/34

(86) Internationale Anmeldenummer:
**PCT/EP1999/004747**

(87) Internationale Veröffentlichungsnummer:
**WO 2000/003976 (27.01.2000 Gazette 2000/04)**

(54) **IMIDAMID-DERIVATE**

IMIDAMIDE DERIVATIVES

DERIVES D'IMIDAMIDE

(84) Benannte Vertragsstaaten:
**AT BE CH CY DE DK ES FI FR GB GR IE IT LI LU MC NL PT SE**

(30) Priorität: **18.07.1998 DE 19832447**
**27.05.1999 DE 19924273**

(43) Veröffentlichungstag der Anmeldung:
**09.05.2001 Patentblatt 2001/19**

(73) Patentinhaber: **Bayer CropScience AG**
**40789 Monheim (DE)**

(72) Erfinder:
- **RIEBEL, Hans-Jochem**
  **D-42113 Wuppertal (DE)**
- **GERDES, Peter**
  **D-52080 Aachen (DE)**
- **GESING, Ernst, Rudolf, F.**
  **D-40699 Erkrath-Hochdahl (DE)**
- **HENSE, Achim**
  **D-42799 Leichlingen (DE)**
- **KANELLAKOPULOS, Johannes**
  **D-41542 Dormagen (DE)**
- **KATHER, Kristian**
  **D-40789 Monheim (DE)**
- **KIRSTEN, Rolf**
  **D-40789 Monheim (DE)**
- **LEHR, Stefan**
  **D-40764 Langenfeld (DE)**
- **ROHE, Lothar**
  **D-42113 Wuppertal (DE)**
- **VOIGT, Katharina**
  **D-52080 Aachen (DE)**
- **WOLLWEBER, Detlef**
  **D-42133 Wuppertal (DE)**
- **ANDERSCH, Wolfram**
  **D-51468 Bergisch Gladbach (DE)**

(56) Entgegenhaltungen:
**EP-A- 0 314 852**
**WO-A-91/04965**
**WO-A-93/04032**
**DE-A- 19 548 783**

- **PATENT ABSTRACTS OF JAPAN vol. 199, no. 711, 28. November 1997 (1997-11-28) & JP 09 194451 A (NIPPON SODA CO LTD), 29. Juli 1997 (1997-07-29)**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 097 128 B1

Printed by Jouve, 75001 PARIS (FR)

## Beschreibung

Die vorliegende Anmeldung betrifft neue Imidamid-Derivate, ein Verfahren zu ihrer Herstellung und ihre Verwendung zur Bekämpfung von tierischen Schädlingen.

Bestimmte Imidamid-Derivate sind bereits bekannt (vgl. WO 91 04 965, WO 93 04 032, EP 0 403 159; J. Organomet. Chem. (1975), 97 (1), S. 39 - 44; Bull. Soc. Chim. Belg. (1981), 90 (1), S 89 - 98). Auch insektizide Eigenschaften einiger dieser Verbindungen waren bekannt.

Es wurden nun neue Imidamid-Derivate der allgemeinen Formel (Ib) gefunden,

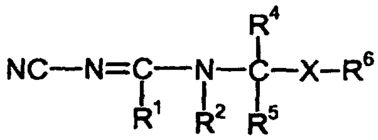

(I b)

in welcher

$R^1$ für Wasserstoff, für gegebenenfalls durch Halogen, Cyano oder $C_1$-$C_4$-Alkoxy substituiertes $C_1$-$C_4$-Alkyl, für gegebenenfalls einfach bis dreifach, gleich oder verschieden durch Halogen oder $C_1$-$C_4$-Alkyl substituiertes $C_3$-$C_6$-Cycloallcyl oder für einfach bis dreifach, gleich oder verschieden substituiertes Phenyl steht, wobei als Substituenten infrage kommen:
Hydroxy, Amino, Cyano, Nitro, Halogen; jeweils gegebenenfalls durch Hydroxy, Cyano oder Halogen substituiertes $C_1$-$C_4$-Alkyl, $C_1$-$C_4$-Alkoxy, $C_1$-$C_4$-Alkylamino und Di-($C_1$-$C_4$-)Alkylamino; jeweils gegebenenfalls durch Halogen substituiertes $C_1$-$C_4$-Alkyl-carbonyl, $C_1$-$C_4$-Alkoxy-carbonyl, $C_1$-$C_4$-Alkyl-carbonyl-amino, $C_1$-$C_4$-Allcylthio, $C_1$-$C_4$-Alkylsulfinyl und $C_1$-$C_4$-Alkylsulfonyl; Aminocarbonyl, Aminothiocarbonyl, $C_1$-$C_4$-Alkylaminocarbonyl, Di-($C_1$-$C_4$)-alkylamino-carbonyl, Aminosulfonyl, $C_1$-$C_4$-Alkylaminosulfonyl und Di-($C_1$-$C_4$)-alkylamino-sulfonyl; sowie jeweils gegebenenfalls durch Hydroxy, Cyano, Nitro, Halogen, $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl und $C_1$-$C_4$-Halogenalkyl, $C_1$-$C_4$-Halogenalkoxy, $C_1$-$C_4$-Halogenalkylthio, $C_1$-$C_4$-Halogenalkylsulfinyl und $C_1$-$C_4$-Halogenalkylsulfonyl mit jeweils 1 bis 5 gleichen oder verschiedenen Halogenatomen aus der Reihe Fluor, Chlor und Brom substituiertes Phenyl, Phenoxy, Phenylthio, Phenylsulfinyl, Phenylsulfonyl, Benzyl und Phenylamino,

$R^2$ für Wasserstoff, für gegebenenfalls durch Halogen, Cyano oder $C_1$-$C_4$-Alkoxy substituiertes $C_1$-$C_4$-Alkyl oder für gegebenenfalls einfach bis dreifach, gleich oder verschieden durch Halogen oder $C_1$-$C_4$-Alkyl substituiertes $C_3$-$C_6$-Cycloalkyl steht und

$R^4$ und $R^5$ unabhängig voneinander für Wasserstoff, für gegebenenfalls durch Halogen, Cyano oder $C_1$-$C_4$-Alkoxy substituiertes $C_1$-$C_4$-Alkyl oder für gegebenenfalls einfach bis dreifach, gleich oder verschieden durch Halogen oder $C_1$-$C_4$-Alkyl substituiertes $C_3$-$C_6$-Cycloalkyl stehen,

$R^6$ für gegebenenfalls einfach bis dreifach, gleich oder verschieden substituiertes Phenyl oder Naphthyl steht, wobei als Substituenten genannt seien:
Cyano, Nitro, Amino, Fluor, Chlor, Brom, Methyl, Ethyl, n- oder i-Propyl, n-, i-, s- oder t-Butyl, Methoxy, Ethoxy, n- oder i-Propoxy, Trifluormethyl, Difluormethyl, Trifluormethoxy, Difluormethoxy, Trifluormethylthio, Methylamino, Dimethylamino, Acetyl, Propionyl, Methoxycarbonyl, Ethoxycarbonyl, Methylthio, Methylsulfinyl, Methylsulfonyl, Methylaminocarbonyl, Ethylaminocarbonyl, n- oder i-Propylaminocarbonyl, Dimethylaminocarbonyl und Diethylaminocarbonyl; sowie jeweils gegebenenfalls durch Cyano, Nitro, Fluor, Chlor, Methyl, Methoxy, Methylthio, Methylsulfinyl, Methylsulfonyl, Trifluormethyl oder Trifluormethoxy, Trifluormethylthio, Trifluormethylsulfinyl oder Trifluormethylsulfonyl substituiertes Phenyl, Phenoxy, Phenylthio, Phenylsulfinyl, Phenylsulfonyl, Benzyl oder Phenylamino,
ferner für jeweils gegebenenfalls einfach bis dreifach, gleich oder verschieden durch Fluor, Chlor, Methyl, Ethyl, i-Propyl, Methoxy, Ethoxy, Trifluormethyl oder Trifluormethoxy substituiertes Cyclopentan oder Cyclohexan steht,
sowie für die folgenden, gegebenenfalls einfach bis fünffach, gleich oder verschieden substituierten Heterocyclen steht:

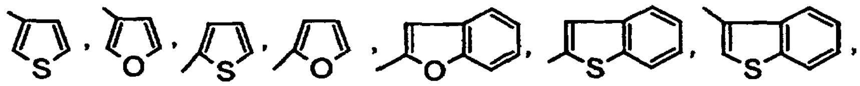
, , , , , , ,

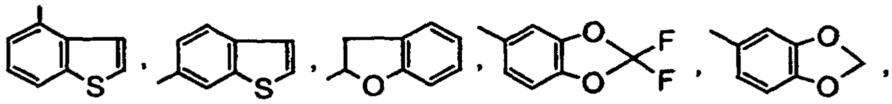
, , , , ,

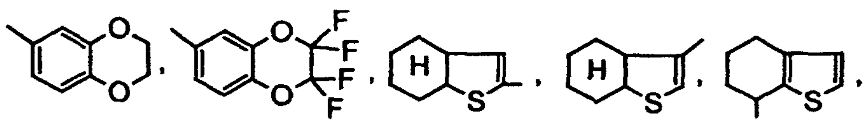
, , , , ,

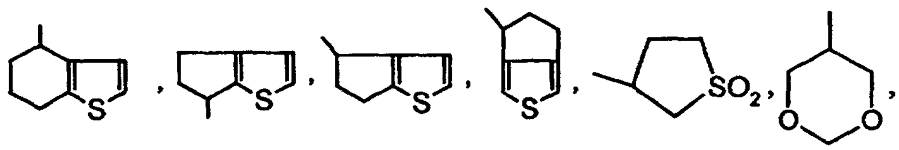
, , , , , ,

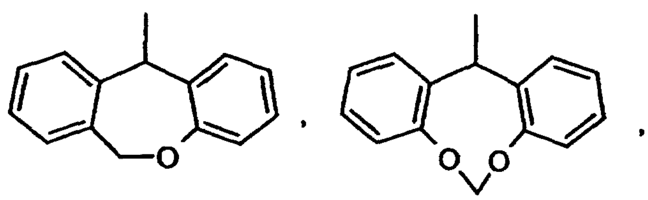
, ,

wobei als Substituenten genannt seien:
Cyano, Nitro, Fluor, Chlor, Brom, Methyl, Ethyl, n- oder i-Propyl, Methoxy, Trifluormethyl oder Dimethylamino oder gegebenenfalls substituiertes Phenyl, wobei als Substituenten für den Phenylrest die Substituenten in Frage kommen, die auch unter $R^6$ für den Phenylrest genannt sind, und

X für eine Einfachbindung sowie für eine der Gruppierungen $-CH_2-$, $-(CH_2)_2-$, $-(CH_2)_3-$, $-CH(CH_3)-$, $-C(CH_3)_2-$, $-CH_2C(CH_3)_2-$, $-CH(CH_3)CH_2-$, $-CH_2O-$, $-CH_2S-$, $-(CH_2)_2O-$, $-(CH_2)_2S-$, $-CH_2OCH_2-$, $CH_2SCH_2-$, $-C(CH_3)=CH$, -C=C-, $-(CH_2)_3-N(CH_3)-$, $-(CH_2)_3-N(C_2H_5)-$, $-(CH_2)_3-N(CH_3)-CH_2-$ oder $-(CH_2)_3-N(C_2H_5)-CH_2-$ steht.

Die Imidamid-Derivate der Formel (Ib) können, auch in Abhängigkeit von den Substituenten, als optische und/oder geometrische Isomere vorkommen. Die vorliegende Erfindung betrifft sowohl die verschiedenen Isomerengemische als auch die reinen Isomeren.

Man erhält die neuen Imidamid-Derivate der allgemeinen Formel (Ib), wenn man Ethanimidsäureester der Formel (II)

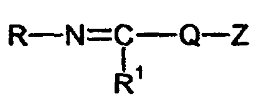
(II)

in welcher

R und $R^1$ die oben angegebene Bedeutung haben,

Q für Sauerstoff oder Schwefel steht und

Z für $C_1$-$C_4$-Alkyl steht,

mit Aminen der Formel (III)

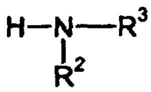 (III)

in welcher

$R^2$ die oben angegebene Bedeutung hat, und

$R^3$ für

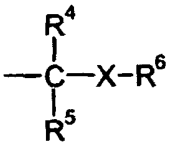

steht, worin $R^4$, $R^5$ und $R^6$ oben angegebene Bedeutung haben,

in Gegenwart eines Verdünnungsmittels.umsetzt.

Die neuen Imidamid-Derivate der allgemeinen Formel (Ib) besitzen stark ausgeprägte biologische Eigenschaften und sind vor allem zur Bekämpfung von tierischen Schädlingen, wie Insekten, Spinnentieren und insbesondere Nematoden, die in der Landwirtschaft, in den Forsten, im Vorrats- und Materialschutz sowie auf dem Hygienesektor vorkommen, geeignet.

Die erfindungsgemäßen Verbindungen sind durch die Formel (Ib) allgemein definiert. Bevorzugte Substituenten bzw. Bereiche der in den oben und nachstehend erwähnten Formeln aufgeführten Reste werden im folgenden erläutert.

$R^1$ steht bevorzugt für Wasserstoff, für jeweils gegebenenfalls durch Cyano, Fluor, Chlor, Methoxy oder Ethoxy substituiertes Methyl, Ethyl, n- oder i-Propyl und n-, i-, s- oder t-Butyl; für jeweils gegebenenfalls einfach bis dreifach, gleich oder verschieden durch Fluor, Chlor, Brom, Methyl oder Ethyl substituiertes Cyclopropyl, Cyclopentyl und Cyclohexyl oder für einfach bis dreifach, gleich oder verschieden substituiertes Phenyl, wobei als Substituenten infrage kommen:
Cyano, Nitro, Amino, Fluor, Chlor, Brom; jeweils gegebenenfalls durch Fluor oder Chlor substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylamino, Ethylamino, n- oder i-Propylamino oder Dimethylamino; jeweils gegebenenfalls durch Fluor und/oder Chlor substituiertes Acetyl, Propionyl, Methoxycarbonyl, Ethoxycarbonyl, n- oder i-Propoxycarbonyl, Methylthio, Methylsulfinyl, Methylsulfonyl, Ethylthio, Ethylsulfinyl oder Ethylsulfonyl; Aminocarbonyl, Aminothiocarbonyl, Methylaminocarbonyl, Ethylaminocarbonyl, n- oder i-Propylaminocarbonyl, Dimethylaminocarbonyl, Diethylaminocarbonyl, Aminosulfonyl, Methylaminosulfonyl, Ethylaminosulfonyl, Dimethylaminosulfonyl und Diethylaminosulfonyl; sowie jeweils gegebenenfalls durch Cyano, Nitro, Fluor, Chlor, Methyl, Ethyl, n- oder i-Propyl, n-, i-, s- oder t-Butyl, Trifluormethyl, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Prapylsulfonyl, Difluormethoxy oder Trifluormethoxy, Trifluormethylthio, Trifluormethylsulfinyl oder Trifluormethylsulfonyl substituiertes Phenyl, Phenoxy, Phenylthio, Phenylsulfinyl, Phenylsulfonyl, Benzyl oder Phenylamino.

$R^2$ steht bevorzugt für Wasserstoff, für jeweils gegebenenfalls durch Cyano, Fluor, Chlor, Methoxy oder Ethoxy substituiertes Methyl, Ethyl, n- oder i-Propyl und n-, i-, s- oder t-Butyl, oder für jeweils gegebenenfalls einfach bis dreifach, gleich oder verschieden durch Fluor, Chlor, Brom, Methyl oder Ethyl substituiertes Cyclopropyl, Cyclopentyl und Cyclohexyl.

$R^4$ und $R^5$ stehen unabhängig voneinander bevorzugt für Wasserstoff, für jeweils gegebenenfalls durch Cyano, Fluor, Chlor, Methoxy oder Ethoxy substituiertes Methyl, Ethyl, n- oder i-Propyl und n-, i-, s- oder t-Butyl, oder für jeweils gegebenenfalls einfach bis dreifach, gleich oder verschieden durch Fluor, Chlor, Brom, Methyl oder Ethyl substituiertes Cyclopropyl, Cyclopentyl und Cyclohexyl.

$R^1$ steht besonders bevorzugt für Wasserstoff, Methyl, Ethyl, n- oder i-Propyl, Cyclopropyl oder Phenyl.

$R^2$ steht besonders bevorzugt für Wasserstoff, Methyl, Ethyl, n- oder i-Propyl oder Cyclopropyl.

$R^4$ und $R^5$ stehen unabhängig voneinander besonders bevorzugt für Wasserstoff, Methyl, Ethyl, n- oder i-Propyl sowie Cyclopropyl.

$R^1 = CH_3$ und

$R^3 =$ 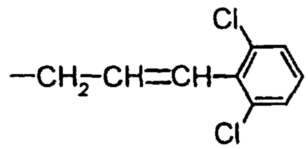

bzw.

$R^1 = C_2H_5$ und

$R^3 =$ 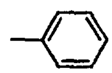

bzw.

$R^1 = CH_3$ und

$R^3 =$ 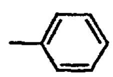

bzw.

$R^1 = i\text{-}C_3H_7$ und

$R^3 =$ 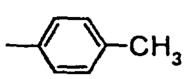

ausgenommen sind.

R steht besonders bevorzugt für Cyano oder Nitro.

$R^1$ steht besonders bevorzugt für Wasserstoff, für jeweils gegebenenfalls durch Cyano, Fluor, Chlor, Methoxy oder Ethoxy substituiertes Methyl, Ethyl, n- oder i-Propyl und n-, i-, s- oder t-Butyl; für jeweils gegebenenfalls einfach bis dreifach, gleich oder verschieden durch Fluor, Chlor, Brom, Methyl oder Ethyl substituiertes Cyclopropyl, Cyclopentyl und Cyclohexyl oder für gegebenenfalls einfach bis dreifach, gleich oder verschieden substituiertes Phenyl, wobei als Substituenten die bei $R^6$ genannten Phenylsubstituenten infrage kommen.

$R^2$ steht besonders bevorzugt für Wasserstoff, für jeweils gegebenenfalls durch Cyano, Fluor, Chlor, Methoxy oder Ethoxy substituiertes Methyl, Ethyl, n- oder i-Propyl und n-, i-, s- oder t-Butyl, oder für jeweils gegebenenfalls einfach bis dreifach, gleich oder verschieden durch Fluor, Chlor, Brom, Methyl oder Ethyl substituiertes Cyclo-

propyl, Cyclopentyl und Cyclohexyl.

$R^3$ steht <u>besonders bevorzugt</u> für die Gruppierungen

Bevorzugte erfindungsgemäße Verbindungen sind auch Stoffe der Formeln (I U) , (I V) und (I W):

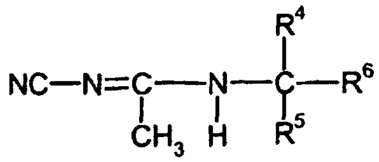

(I U)

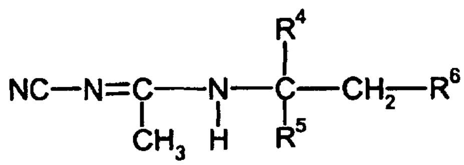

(I V)

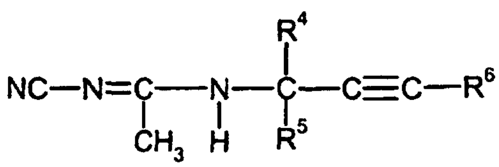

(I W)

in welchen

$R^4$, $R^5$ und $R^6$ für die oben genannten allgemeinen, bevorzugten, oder besonders Bedeutungen stehen.

Bevorzugte erfindungsgemäße Verbindungen sind weiterhin Stoffe der Formeln (I-1) bis (I-23)

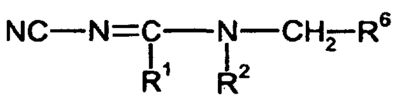

(I-1)

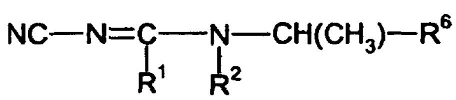

(I-2)

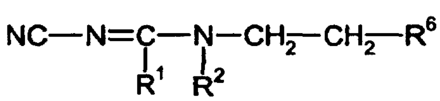

(I-3)

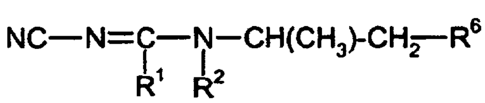

(I-4)

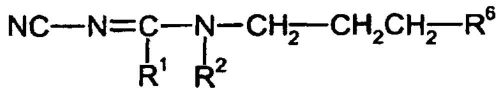

(I-5)

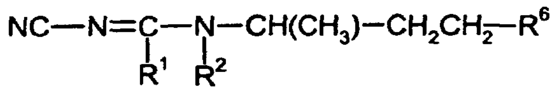

(I-6)

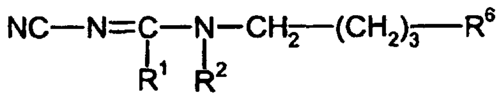

(I-7)

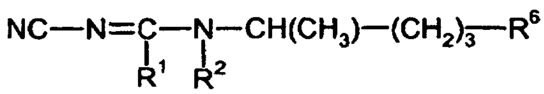

(I-8)

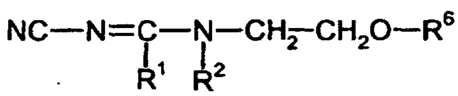

(I-9)

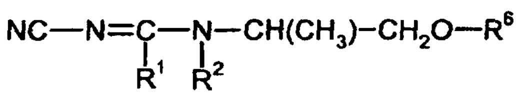

(I-18)

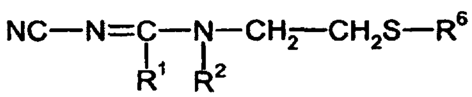

(I-10)

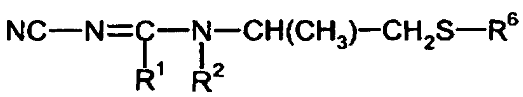

(I-11)

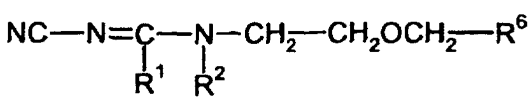

(I-12)

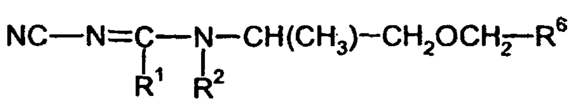

(I-13)

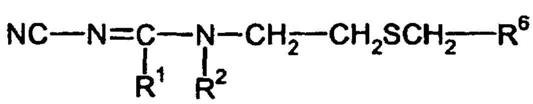

(I-14)

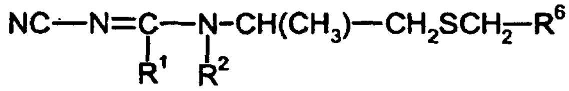

(I-15)

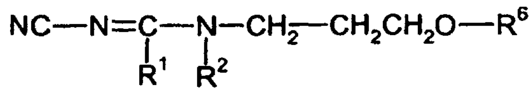

(I-16)

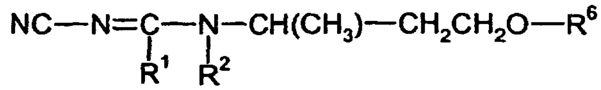

(I-17)

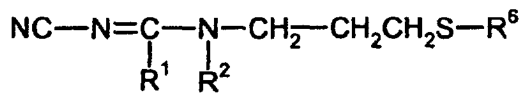

(I-18)

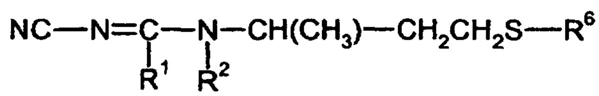

(I-19)

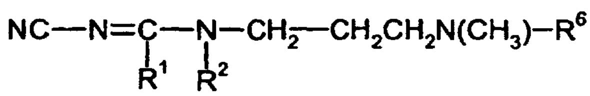

(I-20)

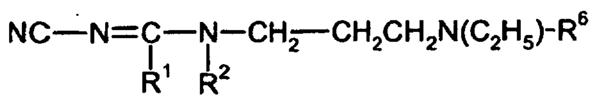

(I-21)

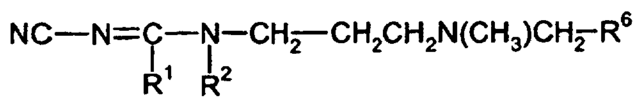

(I-22)

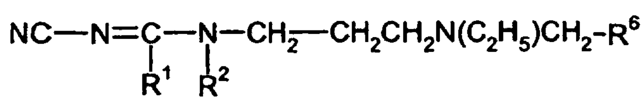

(I-23)

in welchen

$R^1$, $R^2$ und $R^6$ für die oben genannten allgemeinen, bevorzugten und besonders bevorzugten Bedeutungen stehen.

Die oben aufgeführten allgemeinen oder in Vorzugsbereichen aufgeführten Restedefinitionen bzw. Erläuterungen gelten für die Endprodukte und für die Ausgangsund Zwischenprodukte entsprechend. Diese Restedefinitionen können untereinander, also auch zwischen den jeweiligen Vorzugsbereichen, beliebig kombiniert werden.

Erfindungsgemäß bevorzugt werden die Verbindungen der Formel (Ib), in welchen eine Kombination der vorstehend als bevorzugt (vorzugsweise) aufgeführten Bedeutungen vorliegt.

Erfindungsgemäß besonders bevorzugt werden die Verbindungen der Formel (Ib), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

In den oben und nachstehend aufgeführten Restedefinitionen sind Kohlenwasserstoffreste, wie Alkyl oder Alkenyl - auch in Verbindung mit Heteroatomen wie Alkoxy oder Alkylthio - soweit möglich jeweils geradkettig oder verzweigt.

Im einzelnen seien die folgenden Verbindungen genannt:

Tabelle 1

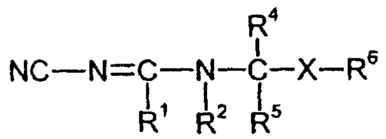

(I b)

Verbindungen der Tabelle 1 entsprechen der allgemeinen Formel (I b), in welcher

$R^1$ = $CH_3$

$R^2$ = H

$C(R^4, R^5)$ = $CH_2$

$XR^6$ = wie im folgenden aufgelistet:

| $XR^6$ | $XR^6$ | $XR^6$ |
|---|---|---|
| 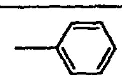 | 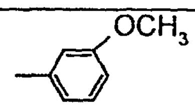 | 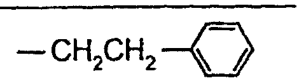 |
| 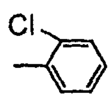 | 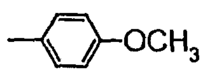 | 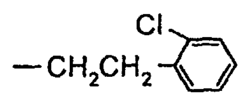 |
| 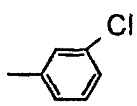 | 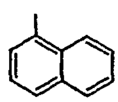 | 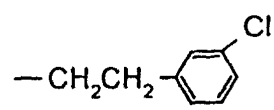 |

Tabelle 1 (Fortsetzung)

| $XR^6$ | $XR^6$ | $XR^6$ |
|---|---|---|
| 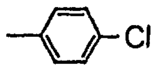 | 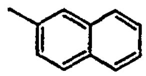 | 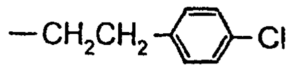 |
| 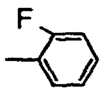 | 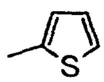 | 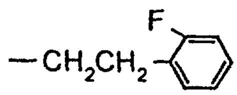 |
| 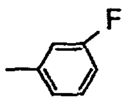 | 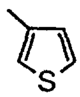 | 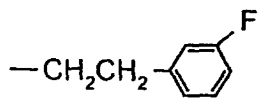 |
| 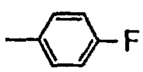 | 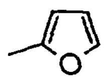 | 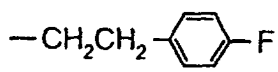 |
| 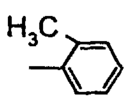 | 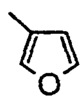 | 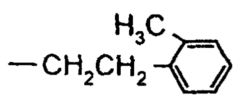 |
| 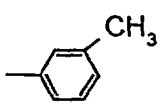 | 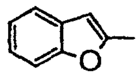 | 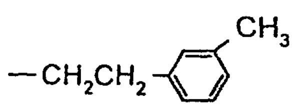 |
| 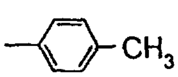 | 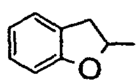 | 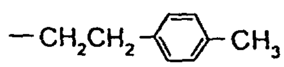 |
| 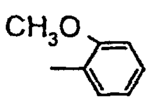 | 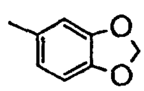 | 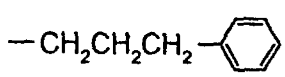 |
| 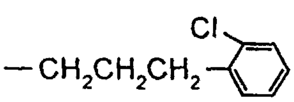 | 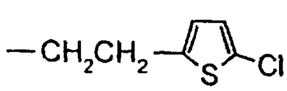 | 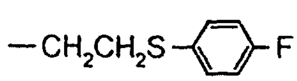 |
| 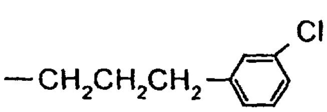 | 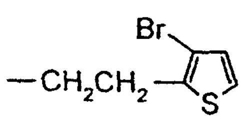 | 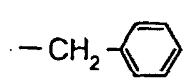 |
| 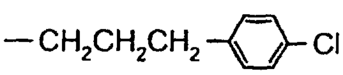 | 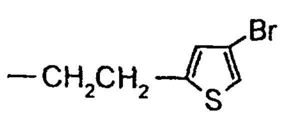 | 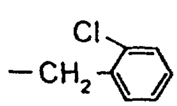 |

Tabelle 1 (Fortsetzung)

| $XR^6$ | $XR^6$ | $XR^6$ |
|---|---|---|
| 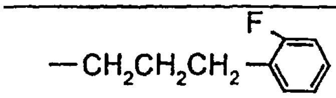 | 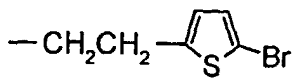 | 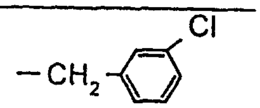 |
| 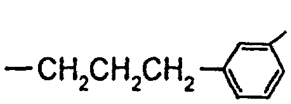 | 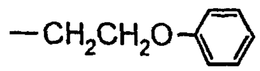 | 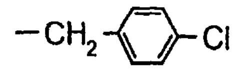 |
| 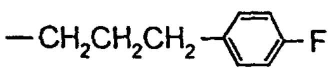 | 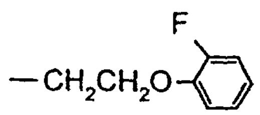 | 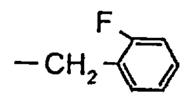 |
| 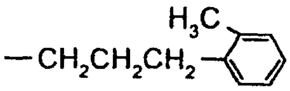 | 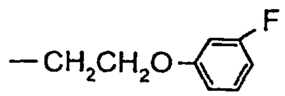 | 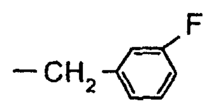 |
| 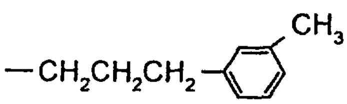 | 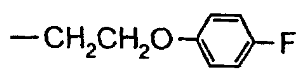 | 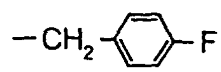 |
| 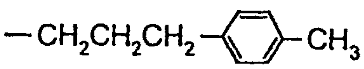 | 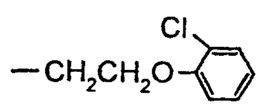 | 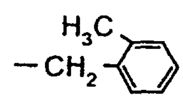 |
| 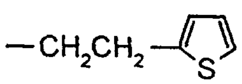 | 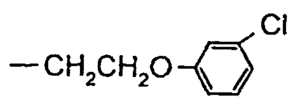 | 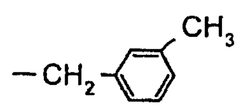 |
| 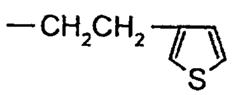 | 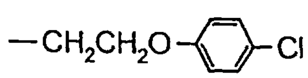 | 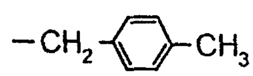 |
| 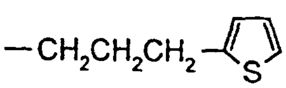 | 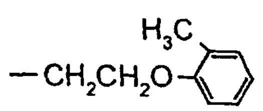 | 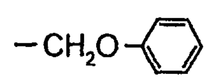 |
| 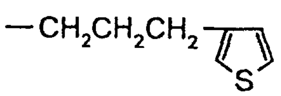 | 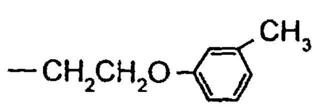 | 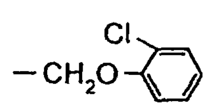 |
| 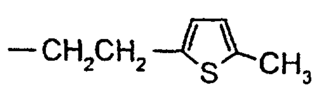 | 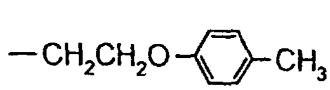 | 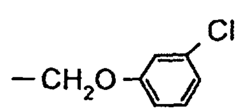 |
| 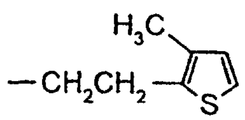 | 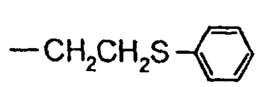 | 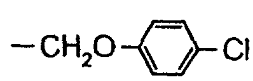 |

Tabelle 1 (Fortsetzung)

| $XR^6$ | $XR^6$ | $XR^6$ |
|---|---|---|
| 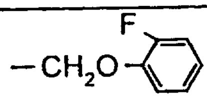 | 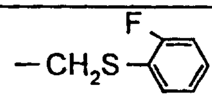 | 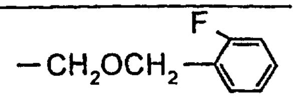 |
| 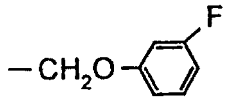 | 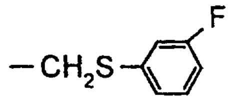 | 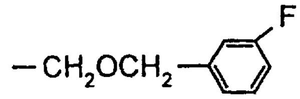 |
| 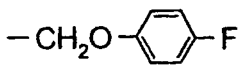 | 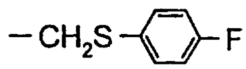 | 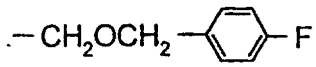 |
| 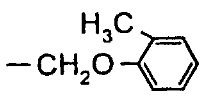 | 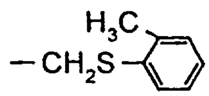 | 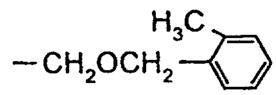 |
| 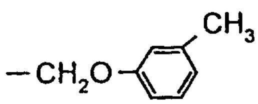 | 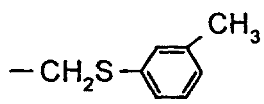 | 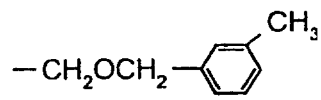 |
| 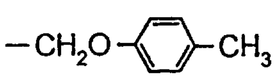 | 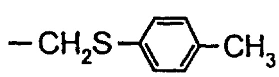 | 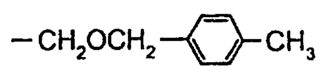 |
| 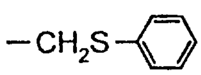 | 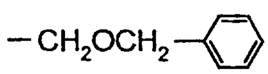 | 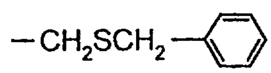 |
| 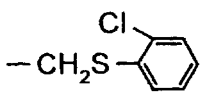 | 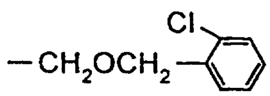 | 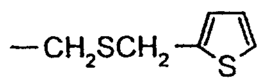 |
| 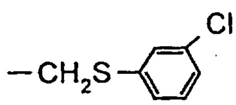 | 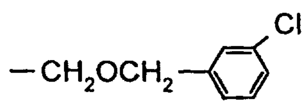 | 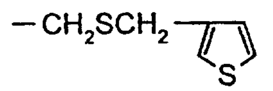 |
| 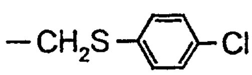 | 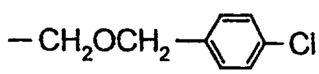 | |

Tabelle 2

Verbindungen der Tabelle 2 entsprechen der allgemeinen Formel (I b), in welcher

| | |
|---|---|
| $C(R^4, R^5)$ | $= CH(CH_3)$ |
| $R^1$, $R^2$ und $XR^6$ | = wie in Tabelle 1 aufgelistet. |

Tabelle 3

Verbindungen der Tabelle 3 entsprechen der allgemeinen Formel (I b), in welcher

| | |
|---|---|
| $C(R^4, R^5)$ | $= CH(C_2H_5)$ |
| $R^1$, $R^2$ und $XR^6$ | = wie in Tabelle 1 aufgelistet. |

Tabelle 4

Verbindungen der Tabelle 4 entsprechen der allgemeinen Formel (I b), in welcher

| | |
|---|---|
| $C(R^4, R^5)$ | $= CH(C_3H_7\text{-i})$ |
| $R^1$, $R^2$ und $XR^6$ | = wie in Tabelle 1 aufgelistet. |

Tabelle 5

Verbindungen der Tabelle 5 entsprechen der allgemeinen Formel (I b), in welcher

| | |
|---|---|
| $C(R^4, R^5)$ | $= CH(C_3H_7\text{-n})$ |
| $R^1$, $R^2$ und $XR^6$ | = wie in Tabelle 1 aufgelistet. |

Tabelle 6

Verbindungen der Tabelle 6 entsprechen der allgemeinen Formel (I b), in welcher

| | |
|---|---|
| $C(R^4, R^5)$ | $= C(CH_3)_2$ |
| $R^1$, $R^2$ und $XR^6$ | = wie in Tabelle 1 aufgelistet. |

Tabellen 7 - 12

Verbindungen der Tabellen 7 - 12 entsprechen der allgemeinen Formel (I b), in welcher

| | |
|---|---|
| $R^1$ | $= C_2H_5$ |
| $R^2$ | = H |
| $C(R^4, R^5)$ | = wie in den Tabellen 1 bis 6 aufgelistet. |
| $XR^6$ | = wie in Tabelle 1 aufgelistet. |

Tabellen 13 - 18

Verbindungen der Tabellen 13 - 18 entsprechen der allgemeinen Formel (I b), in welcher

| | |
|---|---|
| $R^1$ | $= C_3H_7\text{-i}$ |
| $R^2$ | = H |
| $C(R^4, R^5)$ | = wie in den Tabellen 1 bis 6 aufgelistet. |
| $XR^6$ | = wie in Tabelle 1 aufgelistet. |

Tabellen 19 - 24

Verbindungen der Tabellen 19 - 24 entsprechen der allgemeinen Formel (I b), in welcher

| | |
|---|---|
| $R^1$ | $= C_3H_7\text{-n}$ |
| $R^2$ | = H |
| $C(R^4, R^5)$ | = wie in den Tabellen 1 bis 6 aufgelistet. |
| $XR^6$ | = wie in Tabelle 1 aufgelistet. |

Tabellen 25 - 30

Verbindungen der Tabellen 25 - 30 entsprechen der allgemeinen Formel (I b), in welcher

| | | |
|---|---|---|
| $R^1$ | = | 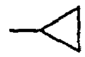 |
| $R^2$ | = | H |
| $C(R^4, R^5)$ | = | wie in den Tabellen 1 bis 6 aufgelistet. |
| $XR^6$ | = | wie in Tabelle 1 aufgelistet. |

Tabellen 31 - 36

Verbindungen der Tabellen 31 - 36 entsprechen der allgemeinen Formel (I b), in welcher

| | |
|---|---|
| $R^1$ | = H |
| $R^2$ | = H |
| $C(R^4, R^5)$ | = wie in den Tabellen 1 bis 6 aufgelistet. |
| $XR^6$ | = wie in Tabelle 1 aufgelistet. |

Tabellen 37 - 42

Verbindungen der Tabellen 37 - 42 entsprechen der allgemeinen Formel (I b), in welcher

| | |
|---|---|
| $R^1$ | = H |
| $R^2$ | = $CH_3$ |
| $C(R^4, R^5)$ | = wie in den Tabellen 1 bis 6 aufgelistet. |
| $XR^6$ | = wie in Tabelle 1 aufgelistet. |

Tabellen 43 - 48

Verbindungen der Tabellen 43 -48 entsprechen der allgemeinen Formel (I b), in welcher

| | |
|---|---|
| $R^1$ | = H |
| $R^2$ | = $C_2H_5$ |
| $C(R^4, R^5)$ | = wie in den Tabellen 1 bis 6 aufgelistet. |
| $XR^6$ | = wie in Tabelle 1 aufgelistet. |

Tabellen 49 - 54

Verbindungen der Tabellen 49 -54 entsprechen der allgemeinen Formel (I b), in welcher

| | |
|---|---|
| $R^1$ | = $CH_3$ |
| $R^2$ | = $CH_3$ |
| $C(R^4, R^5)$ | = wie in den Tabellen 1 bis 6 aufgelistet. |
| $XR^6$ | = wie in Tabelle 1 aufgelistet. |

Tabellen 55 - 60

Verbindungen der Tabellen 55 -60 entsprechen der allgemeinen Formel (I b), in welcher

| | |
|---|---|
| $R^1$ | = $CH_3$ |

(fortgesetzt)

| | |
|---|---|
| $R^2$ | $= C_2H_5$ |
| $C(R^4, R^5)$ | = wie in den Tabellen 1 bis 6 aufgelistet. |
| $XR^6$ | = wie in Tabelle 1 aufgelistet. |

Tabellen 61 - 66

Verbindungen der Tabellen 61 -66 entsprechen der allgemeinen Formel (I b), in welcher

| | |
|---|---|
| $R^1$ | $= CH_3$ |
| $R^2$ | $= C_3H_7$-i |
| $C(R^4, R^5)$ | = wie in den Tabellen 1 bis 6 aufgelistet. |
| $XR^6$ | = wie in Tabelle 1 aufgelistet. |

Tabellen 67 - 72

Verbindungen der Tabellen 67 -72 entsprechen der allgemeinen Formel (I b), in welcher

| | |
|---|---|
| $R^1$ | $= C_2H_5$ |
| $R^2$ | $= CH_3$ |
| $C(R^4, R^5)$ | = wie in den Tabellen 1 bis 6 aufgelistet. |
| $XR^6$ | = wie in Tabelle 1 aufgelistet. |

Tabellen 73 - 78

Verbindungen der Tabellen 73 -78 entsprechen der allgemeinen Formel (I b), in welcher

| | |
|---|---|
| $R^1$ | $= C_2H_5$ |
| $R^2$ | $= C_2H_5$ |
| $C(R^4, R^5)$ | = wie in den Tabellen 1 bis 6 aufgelistet. |
| $XR^6$ | = wie in Tabelle 1 aufgelistet. |

Tabellen 79 - 84

Verbindungen der Tabellen 79 - 84 entsprechen der allgemeinen Formel (I b), in welcher

| | |
|---|---|
| R' | $= C_3H_7$-i |
| $R^2$ | $= CH_3$ |
| $C(R^4, R^5)$ | = wie in den Tabellen 1 bis 6 aufgelistet. |
| $XR^6$ | = wie in Tabelle 1 aufgelistet. |

Tabellen 85 - 90

Verbindungen der Tabellen 85 -90 entsprechen der allgemeinen Formel (I b), in welcher

| | |
|---|---|
| $R^1$ | $= C_3H_7$-i |
| $R^2$ | $= C_2H_5$ |
| $C(R^4, R^5)$ | = wie in den Tabellen 1 bis 6 aufgelistet. |
| $XR^6$ | = wie in Tabelle 1 aufgelistet. |

Tabellen 91 - 96

Verbindungen der Tabellen 91 - 96 entsprechen der allgemeinen Formel (I b), in welcher

| | |
|---|---|
| $R^1$ | = $C_3H_7$-n |
| $R^2$ | = $CH_3$ |
| $C(R^4, R^5)$ | = wie in den Tabellen 1 bis 6 aufgelistet. |
| $XR^6$ | = wie in Tabelle 1 aufgelistet. |

Tabellen 97 - 102

Verbindungen der Tabellen 97 - 102 entsprechen der allgemeinen Formel (I b), in welcher

| | |
|---|---|
| $R^1$ | = $C_3H_7$-n |
| $R^2$ | = $C_2H_5$ |
| $C(R^4, R^5)$ | = wie in den Tabellen 1 bis 6 aufgelistet. |
| $XR^6$ | = wie in Tabelle 1 aufgelistet. |

Tabellen 103 - 108

Verbindungen der Tabellen 103 - 108 entsprechen der allgemeinen Formel (I b), in welcher

| | | |
|---|---|---|
| $R^1$ | = | 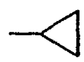 |
| $R^2$ | = | $CH_3$ |
| $C(R^4, R^5)$ | = | wie in den Tabellen 1 bis 6 aufgelistet. |
| $XR^6$ | = | wie in Tabelle 1 aufgelistet. |

Tabellen 109 - 114

Verbindungen der Tabellen 109 -114 entsprechen der allgemeinen Formel (I b), in welcher

| | | |
|---|---|---|
| $R^1$ | = | 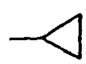 |
| $R^2$ | = | $C_2H_5$ |
| $C(R^4, R^5)$ | = | wie in den Tabellen 1 bis 6 aufgelistet. |
| $XR^6$ | = | wie in Tabelle 1 aufgelistet. |

Bevorzugt genannt seien die folgenden Verbindungen:

Tabelle a

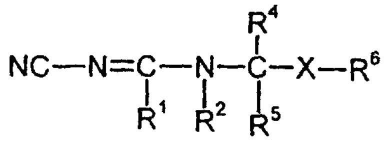 (I b)

| $R^1$ | $R^2$ | $R^4$ | $R^5$ | X | $R^6$ |
|---|---|---|---|---|---|
| $CH_3$ | H | H | $CH_3$ | $-CH_2CH_2-$ | 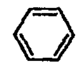 |
| $CH_3$ | H | H | $CH_3$ | $-CH_2CH_2-$ | 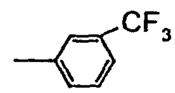 |
| $CH_3$ | H | H | $CH_3$ | $-CH_2CH_2-$ | 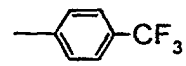 |
| $CH_3$ | H | H | $CH_3$ | $-CH_2CH_2-$ | 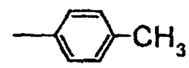 |
| $CH_3$ | H | H | $CH_3$ | $-CH_2CH_2-$ | 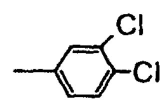 |
| $CH_3$ | H | H | $CH_3$ | $-CH_2CH_2-$ | 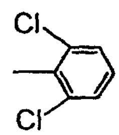 |
| $CH_3$ | H | $CH_3$ | $CH_3$ | $-CH_2CH_2-$ | 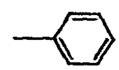 |
| $CH_3$ | H | $CH_3$ | $CH_3$ | $-CH_2CH_2-$ | 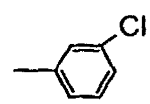 |
| $CH_3$ | H | $CH_3$ | $CH_3$ | $-CH_2CH_2-$ | 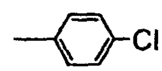 |
| $CH_3$ | H | $CH_3$ | $CH_3$ | $-CH_2CH_2-$ | 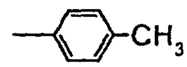 |
| $CH_3$ | H | $CH_3$ | $CH_3$ | $-CH_2CH_2-$ | 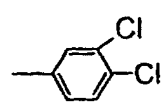 |
| $CH_3$ | H | $CH_3$ | $CH_3$ | $-CH_2CH_2-$ | 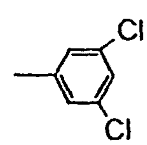 |
| $CH_3$ | H | H | $CH_3$ | $-CH(CH_3)CH_2-$ | 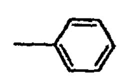 |

Tabelle a (Fortsetzung)

| $R^1$ | $R^2$ | $R^4$ | $R^5$ | X | $R^6$ |
|---|---|---|---|---|---|
| $CH_3$ | H | H | $CH_3$ | $-CH(CH_3)CH_2-$ | 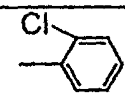 |
| $CH_3$ | H | H | $CH_3$ | $-CH(CH_3)CH_2-$ | 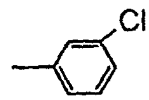 |
| $CH_3$ | H | H | $CH_3$ | $-CH(CH_3)CH_2-$ | 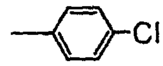 |
| $CH_3$ | H | H | $CH_3$ | $-CH(CH_3)CH_2-$ | 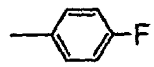 |
| $CH_3$ | H | H | $CH_3$ | $-CH(CH_3)CH_2-$ | 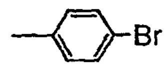 |
| $CH_3$ | H | H | $CH_3$ | $-CH(CH_3)CH_2-$ | 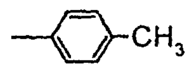 |
| $CH_3$ | H | H | $CH_3$ | $-CH(CH_3)CH_2-$ | 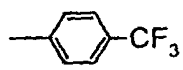 |
| $CH_3$ | H | H | H | $-CH(CH_3)CH_2-$ | 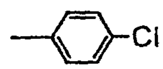 |
| $CH_3$ | H | H | H | $-CH(CH_3)CH_2-$ | 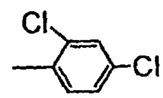 |
| $CH_3$ | H | H | $CH_3$ | $-(CH_2)_3N(CH_3)-$ | 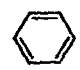 |
| $CH_3$ | H | H | $CH_3$ | $-(CH_2)_3N(C_2H_5)-$ | 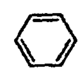 |
| $CH_3$ | H | H | H | $-CH(CH_3)-$ | 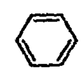 |
| $CH_3$ | H | H | H | $-CH(CH_3)-$ | 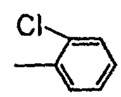 |
| $CH_3$ | H | H | H | $-CH(CH_3)-$ | 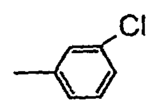 |
| $CH_3$ | H | H | H | $-CH(CH_3)-$ | 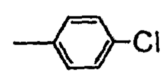 |
| $CH_3$ | H | H | H | $-CH(CH_3)-$ | 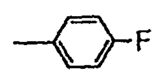 |
| $CH_3$ | H | H | H | $-CH(CH_3)-$ | 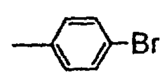 |
| $CH_3$ | H | H | H | $-CH(CH_3)-$ | 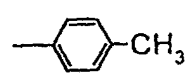 |

Tabelle a (Fortsetzung)

| $R^1$ | $R^2$ | $R^4$ | $R^5$ | X | $R^6$ |
|---|---|---|---|---|---|
| $CH_3$ | H | H | H | $-CH(CH_3)-$ | 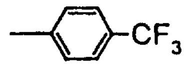 |
| $CH_3$ | H | $CH_3$ | $CH_3$ | -C≡C- | 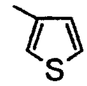 |
| $CH_3$ | H | H | $CH_3$ | - | 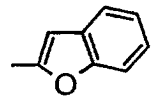 |
| $CH_3$ | H | H | $CH_3$ | - | 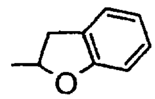 |
| $CH_3$ | H | H | $CH_3$ | - | 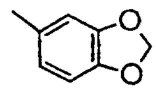 |
| $CH_3$ | H | H | $CH_3$ | - | 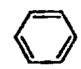 |
| $CH_3$ | H | H | $CH_3$ | - | 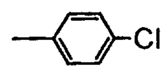 |
| $CH_3$ | H | H | $n-C_3H_7$ | - | 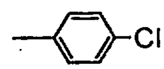 |
| $CH_3$ | H | H | H | $-C(CH_3)=CH-$ | 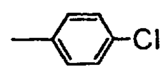 |
| $CH_3$ | H | H | $CH_3$ | $-CH_2O-$ | 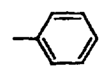 |
| $CH_3$ | H | H | $CH_3$ | $-CH_2O-$ | 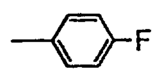 |
| $CH_3$ | H | H | $CH_3$ | $-CH_2O-$ | 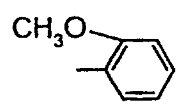 |
| $CH_3$ | H | H | $CH_3$ | $-CH_2O-$ | 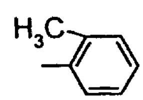 |
| $CH_3$ | H | H | $CH_3$ | $-CH_2O-$ | 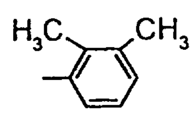 |
| $CH_3$ | H | H | $CH_3$ | $-CH_2O-$ | 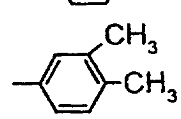 |
| $CH_3$ | H | H | $CH_3$ | $-CH_2O-$ | 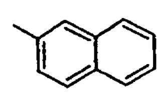 |
| $CH_3$ | H | $CH_3$ | $CH_3$ | -C≡C- | 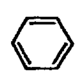 |

Tabelle a (Fortsetzung)

| $R^1$ | $R^2$ | $R^4$ | $R^5$ | X | $R^6$ |
|---|---|---|---|---|---|
| $CH_3$ | H | $CH_3$ | $CH_3$ | -C≡C- | 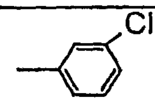 |
| $CH_3$ | H | $CH_3$ | $CH_3$ | -C≡C- | 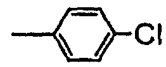 |
| $CH_3$ | H | $CH_3$ | $CH_3$ | -C≡C- | 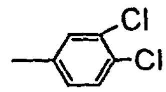 |
| $CH_3$ | H | $CH_3$ | $CH_3$ | -C≡C- | 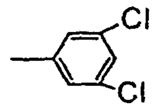 |
| $CH_3$ | H | $CH_3$ | $CH_3$ | -C≡C- | 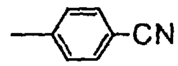 |
| $CH_3$ | H | $CH_3$ | $CH_3$ | -C≡C- | 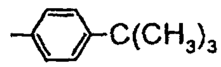 |
| $CH_3$ | H | $CH_3$ | $CH_3$ | -C≡C- | 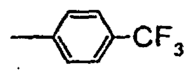 |
| $CH_3$ | H | $CH_3$ | $CH_3$ | -C≡C- | 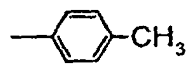 |
| $CH_3$ | H | $CH_3$ | $CH_3$ | -C≡C- | 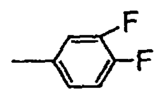 |
| $CH_3$ | H | H | $CH_3$ | $-CH_2-$ | 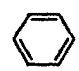 |
| $CH_3$ | H | H | $CH_3$ | $-CH_2-$ | 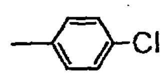 |

Tabelle b

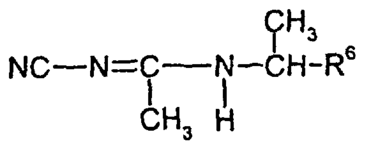

(I b-1)

| $R^6$ | $R^6$ | $R^6$ | $R^6$ |
|---|---|---|---|
| 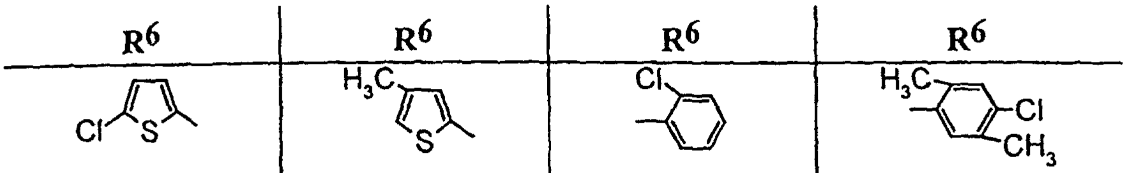 | | | |
| 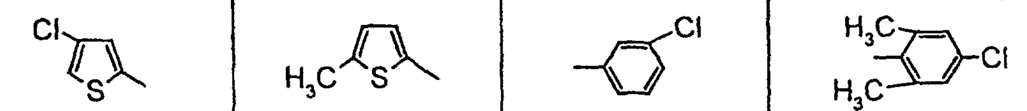 | | | |
| 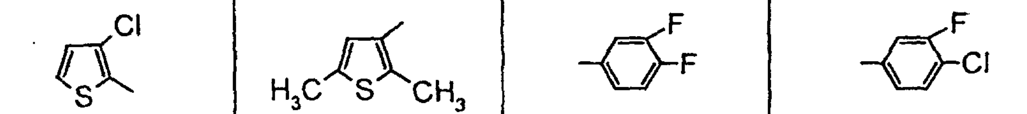 | | | |
| 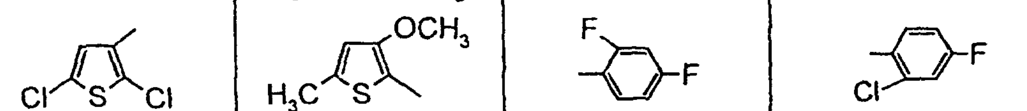 | | | |
| 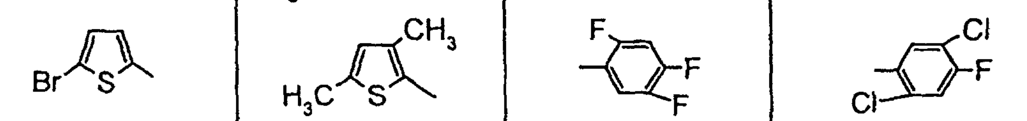 | | | |
| 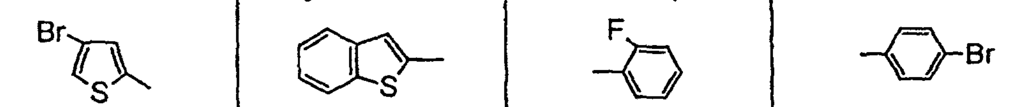 | | | |
| 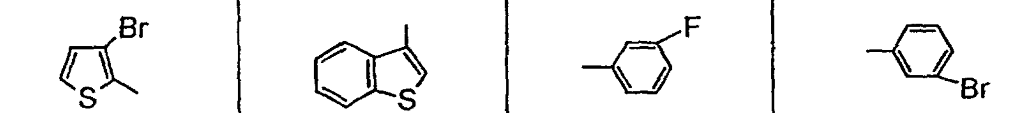 | | | |
| 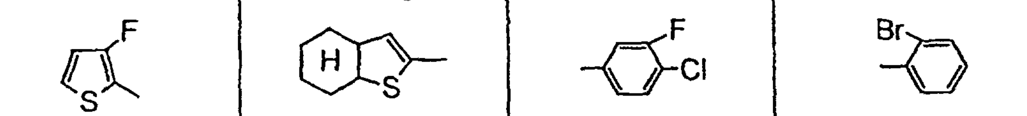 | | | |
| 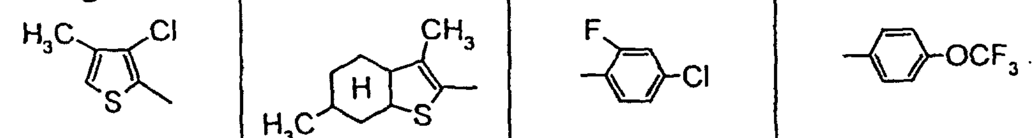 | | | |
| 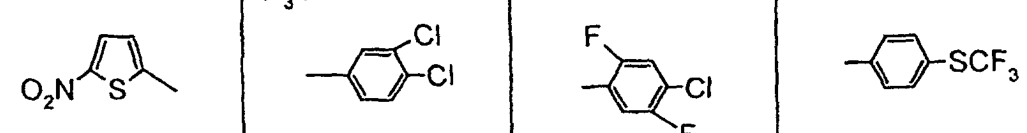 | | | |
| 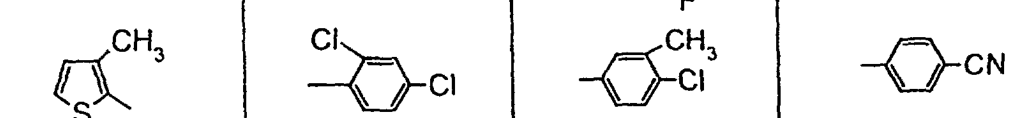 | | | |
| 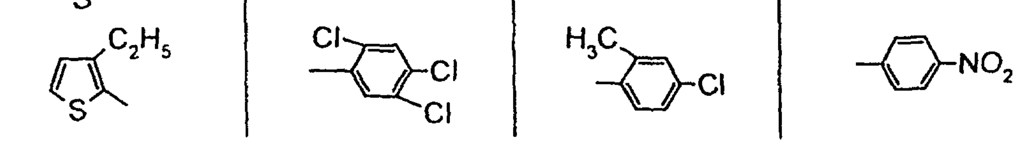 | | | |

Tabelle b (Fortsetzung)

| $R^6$ | $R^6$ | $R^6$ | $R^6$ |
|---|---|---|---|
| 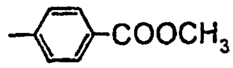 | 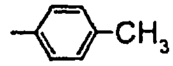 | 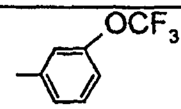 | 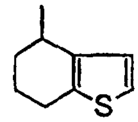 |
| 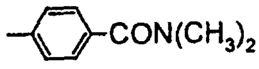 | 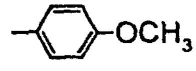 | 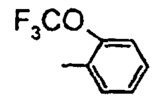 | 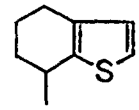 |
| 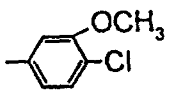 | 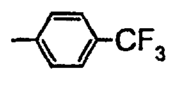 | 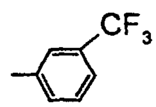 | 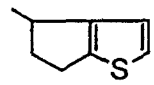 |
| 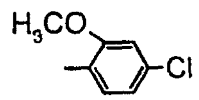 | 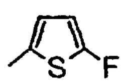 | 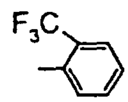 | 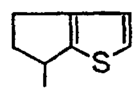 |
| 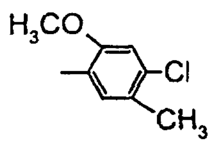 | 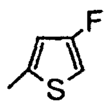 | | |

Tabelle c

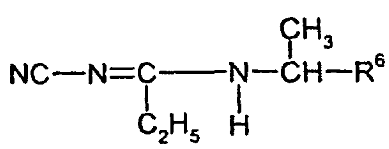

(I b-2)

$R^6$ = wie in Tabelle b aufgeführt, sowie zusätzlich

| $R^6$ | $R^6$ | $R^6$ | $R^6$ |
|---|---|---|---|
| 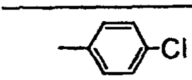 | 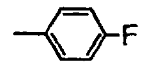 | 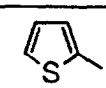 | 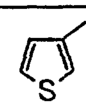 |

Tabelle d

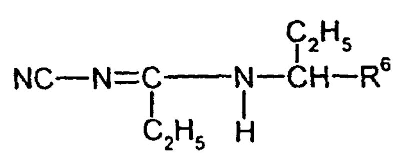

(I b-3)

$R^6$ = wie in Tabelle c aufgeführt.

Tabelle e

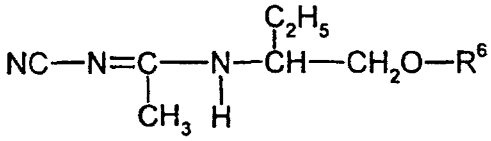

(I b-4)

| $R^6$ | $R^6$ | $R^6$ | $R^6$ | $R^6$ |
|---|---|---|---|---|
| 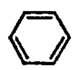 | 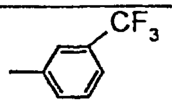 | 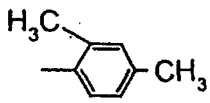 | 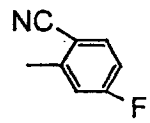 | 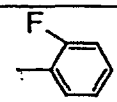 |
| 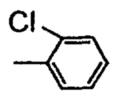 | 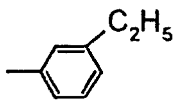 | 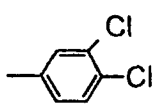 | 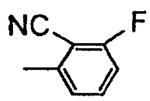 | 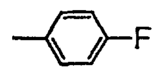 |
| 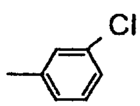 | 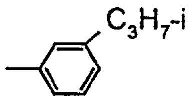 | 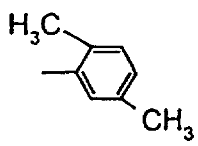 | 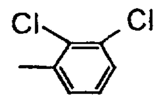 | 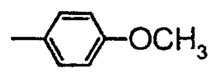 |
| 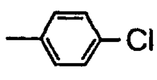 | 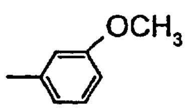 | 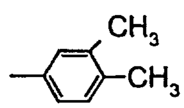 | 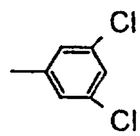 | |
| 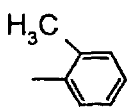 | 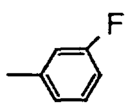 | 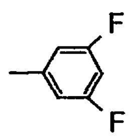 | 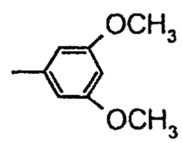 | |
| 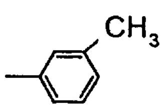 | 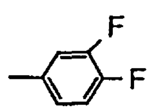 | 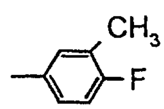 | 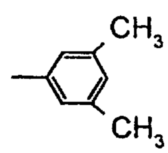 | |
| 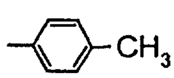 | 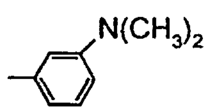 | 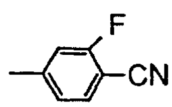 | 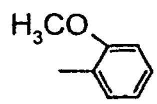 | |

Tabelle f

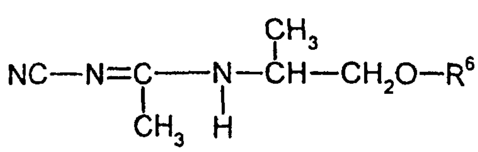

(I b-5)

$R^6$ = wie in Tabelle e aufgeführt.

Tabelle g

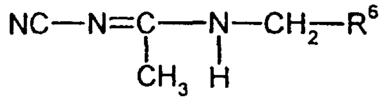 (I b-6)

| $R^6$ | $R^6$ | $R^6$ |
|---|---|---|
| 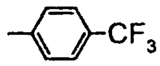 | 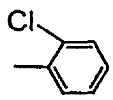 | 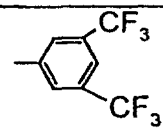 |
| 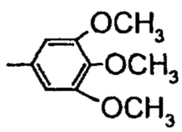 | 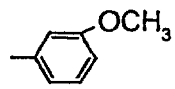 | 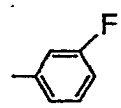 |
| 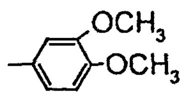 | 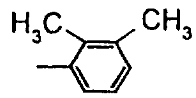 | 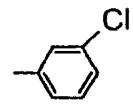 |
| 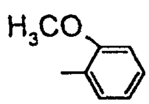 | 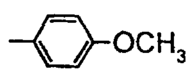 | 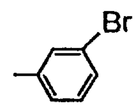 |
| 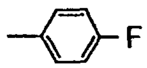 | 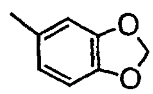 | 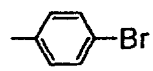 |
| 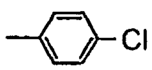 | 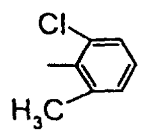 | 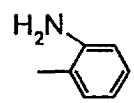 |
| 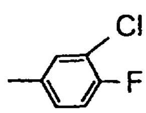 | 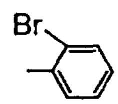 | 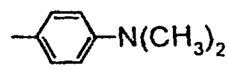 |
| 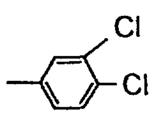 | 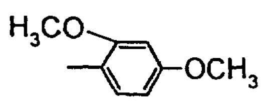 | 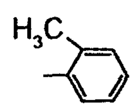 |

Tabelle g (Fortsetzung)

| $R^6$ | $R^6$ | $R^6$ |
|---|---|---|
| 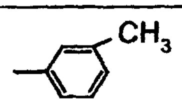 | 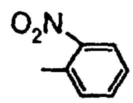 | 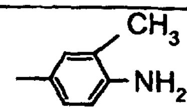 |
| 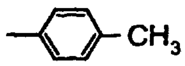 | 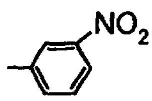 | 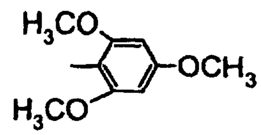 |
| 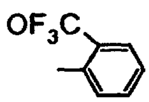 | 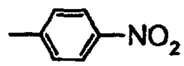 | 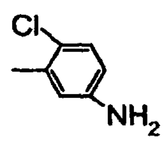 |
| 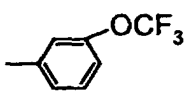 | 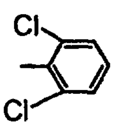 | 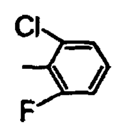 |
| 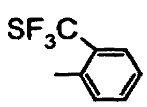 | 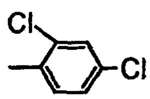 | 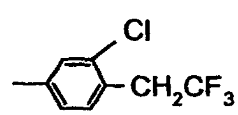 |
| 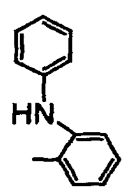 | 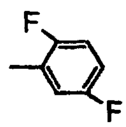 | 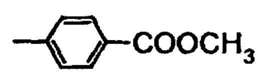 |
| 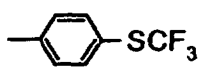 | 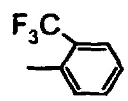 | 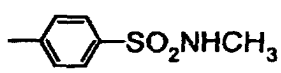 |

Die o.g. erfindungsgemäßen Verbindungen der verschiedenen Formeln und in den einzelnen Tabellen können gegebenenfalls als Racemate, R- oder S-Isomere vorliegen. Verwendet man beispielsweise N-Cyano-ethanimidsäuremethylester und 4-Chlor-2-methylbenzylamin als Ausgangsstoffe, so kann der Verlauf des erfindungsgemäßen Verfahrens durch das folgende Reaktionsschema wiedergegeben werden:

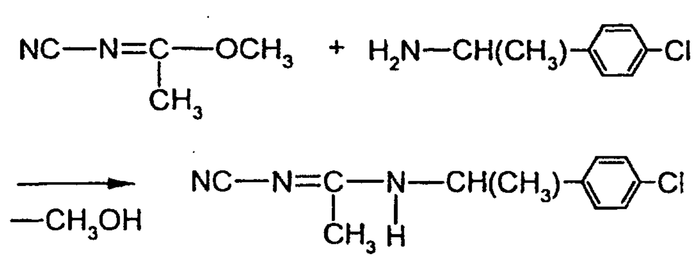


Die zur Durchführung des erfindungsgemäßen Verfahrens als Ausgangsstoffe benötigten Ethanimidsäureester sind durch die Formel (II) allgemein definiert. In dieser Formel steht Z vorzugsweise für $C_1$-$C_4$-Alkyl, wie insbesondere für Methyl oder Ethyl.

Die N-Cyano-ethanimidsäureester sind weitgehend bekannt (vgl. z.B. US 5.304 566 oder J. Org. Chem. 28,

1963, 1816-1821) und/oder nach üblichen Verfahren erhältlich.

Die N-Nitro-ethanimidsäureester werden erhalten indem man z.B. die entsprechenden $NO_2$-freien Ethanimidsäureester der Formel (II) in üblicher Weise nitriert.

Die weiterhin beim erfindungsgemäßen Verfahren als Ausgangsstoffe zu verwendenden Amine der allgemeinen Formel (III) sind allgemein bekannte Verbindungen der organischen Chemie bzw. in allgemein bekannter Art und Weise erhältlich.

Das erfindungsgemäße Verfahren wird vorzugsweise in Gegenwart eines Verdünnungsmittels durchgeführt. Vorzugsweise verwendbar sind Alkohole, wie Methanol und Ethanol; Nitrile, wie Acetonitril oder Ester, wie Essigsäureethylester. Es ist auch möglich, gegebenenfalls in Wasser oder organisch-wässrigen Gemischen zu arbeiten.

Bei der Durchführung des erfindungsgemäßen Verfahrens arbeitet man vorzugsweise in äquimolaren Mengen; es ist aber auch möglich, das eine oder andere Ausgangsprodukt im Überschuß einzusetzen.

Die Reaktionstemperaturen können bei der Durchführung des erfindungsgemäßen Verfahrens in einem größeren Bereich variiert werden. Im allgemeinen arbeitet man bei Temperaturen zwischen 0°C und 100°C, bevorzugt zwischen 20°C und 80°C.

Aufarbeitung und Isolierung der Endprodukte erfolgen in allgemein bekannter Art und Weise.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.

Aus der Ordnung der Chilopoda z.B. Geophilus carpophagus, Scutigera spp..

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Acheta domesticus, Gryllotalpa spp., Locusta migratoria migratorioides, Melanoplus spp., Schistocerca gregaria.

Aus der Ordnung der Blattaria z.B. Blatta orientalis, Periplaneta americana, Leucophaea maderae, Blattella germanica.

Aus der Ordnung der Dermaptera z.B. Forficula auricularia.

Aus der Ordnung der Isoptera z.B. Reticulitermes spp..

Aus der Ordnung der Phthiraptera z.B. Pediculus humanus corporis, Haematopinus spp., Linognathus spp., Trichodectes spp., Damalinia spp..

Aus der Ordnung der Thysanoptera z.B. Hercinothrips femoralis, Thrips tabaci, Thrips palmi, Frankliniella accidentalis.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium comi, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella xylustella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis,

Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..

Aus der Ordnung der Siphonaptera z.B. Xenopsylla cheopis, Ceratophyllus spp..

Aus der Klasse der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursa phelenchus spp..

Die erfindungsgemäßen Verbindungen der Formel (Ib) zeichnen sich insbesondere durch eine gute nematizide Wirksamkeit aus. So lassen sie sich beispielsweise mit besonders gutem Erfolg zur Bekämpfung von Meloidogyne incognita einsetzen

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

In den entsprechenden Aufwandmengen zeigen die erfindungsgemäßen Verbindungen teilweise herbizide Wirkungen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

**Fungizide:**

Aldimorph, Ampropylfos, Ampropylfos-Kalium, Andoprim, Anilazin, Azaconazol, Azoxystrobin,

Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,

Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,

Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,

Ediphenphos, Epoxiconazol, Etaconazol, Ethirimol, Etridiazol,

Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,

Guazatin,

Hexachlorobenzol, Hexaconazol, Hymexazol,

Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,

Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,

Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,

Nickel-dimethyldithiocarbamat, Nitrothal-isopropyl, Nuarimol,

Ofurace, Oxadixyl, Oxamocarb, Oxolinicacid, Oxycarboxim, Oxyfenthiin,

Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,

Quinconazol, Quintozen (PCNB),

Schwefel und Schwefel-Zubereitungen,

Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,

Uniconazol,

Validamycin A, Vinclozolin, Viniconazol,

Zarilamid, Zineb, Ziram sowie

Dagger G,

OK-8705,

OK-8801,

α-(1,1-Dimethylethyl)-β-(2-phenoxyethyl)-1H-1,2,4-triazol-1-ethanol,

α-(2,4-Dichlorphenyl)-β-fluor-b-propyl-1H-1,2,4-triazol-1-ethanol,

α-(2,4-Dichlorphenyl)-β-methoxy-a-methyl-1H-1,2,4-triazol-1-ethanol,

α-(5-Methyl-1,3-dioxan-5-yl)-β-[[4-(trifluormethyl)-phenyl]-methylen]-1H-1,2,4-triazol-1-ethanol,

(5RS,6RS)-6-Hydroxy-2,2,7,7-tetramethyl-5-(1H-1,2,4-triazol-1-yl)-3-octanon,

(E)-a-(Methoxyimino)-N-methyl-2-phenoxy-phenylacetamid,

{2-Methyl-1-[[[1-(4-methylphenyl)-ethyl]-amino]-carbonyl]-propyl}-carbaminsäure-1-isopropylester

1-(2,4-Dichlorphenyl)-2-(1H-1,2,4-triazol-1-yl)-ethanon-O-(phenylmethyl)-oxim,

1-(2-Methyl-1-naphthalenyl)-1H-pyrrol-2,5-dion,

1-(3,5-Dichlorphenyl)-3-(2-propenyl)-2,5-pyrrolidindion,

1-[(Diiodmethyl)-sulfonyl]-4-methyl-benzol,

1-[[2-(2,4-Dichlorphenyl)-1,3-dioxolan-2-yl]-methyl]-1H-imidazol,

1-[[2-(4-Chlorphenyl)-3-phenyloxiranyl]-methyl]-1H-1,2,4-triazol,

1-[ 1-[2-[(2,4-Dichlorphenyl)-methoxy]-phenyl]-ethenyl]-1H-imidazol,

1-Methyl-5-nonyl-2-(phenylmethyl)-3-pyrrolidinol,

2',6'-Dibrom-2-methyl-4'-trifluormethoxy-4'-trifluor-methyl-1,3-thiazol-5-carboxanilid,

2,2-Dichlor-N-[1-(4-chlorphenyl)-ethyl]-1-ethyl-3-methyl-cyclopropancarboxamid,

2,6-Dichlor-5-(methylthio)-4-pyrimidinyl-thiocyanat,

2,6-Dichlor-N-(4-trifluormethylbenzyl)-benzamid,

2,6-Dichlor-N-[[4-(trifluormethyl)-phenyl]-methyl]-benzamid,

2-(2,3,3-Triiod-2-propenyl)-2H-tetrazol,

2-[(1-Methylethyl)-sulfonyl]-5-(trichlormethyl)-1,3,4-thiadiazol,

2-[[6-Deoxy-4-O-(4-O-methyl-β-D-glycopyranosyl)-a-D-glucopyranosyl]-amino]-4-methoxy-1H-pyrrolo[2,3-d] pyrimidin-5-carbonitril,

2-Aminobutan,

2-Brom-2-(brommethyl)-pentandinitril,

2-Chlor-N-(2,3-dihydro-1,1,3-trimethyl-1H-inden-4-yl)-3-pyridincarboxamid,

2-Chlor-N-(2,6-dimethylphenyl)-N-(isothiocyanatomethyl)-acetamid,

2-Phenylphenol(OPP),

3,4-Dichlor-1-[4-(difluormethoxy)-phenyl]-1H-pyrrol-2,5-dion,

3,5-Dichlor-N-[cyan[(1-methyl-2-propynyl)-oxy]-methyl]-benzamid,

3-(1,1-Dimethylpropyl-1-oxo-1H-inden-2-carbonitril,

3-[2-(4-Chlorphenyl)-5-ethoxy-3-isoxazolidinyl]-pyridin,

4-Chlor-2-cyan-N,N-dimethyl-5-(4-methylphenyl)-1H-imidazol-1-sulfonamid,

4-Methyl-tetrazolo[1,5-a]quinazolin-5(4H)-on,

8-(1,1-Dimethylethyl)-N-ethyl-N-propyl-1,4-dioxaspiro[4.5]decan-2-methanamin,

8-Hydroxychinolinsulfat,

9H-Xanthen-9-carbonsäure-2-[(phenylamino)-carbonyl]-hydrazid,

bis-(1-Methylethyl)-3-methyl-4-[(3-methylbenzoyl)-oxy]-2,5-thiophendicarboxylat,

cis-1-(4-Chlorphenyl)-2-(1H-1,2,4-triazol-1-yl)-cycloheptanol,

cis-4-[3-[4-(1,1-Dimethylpropyl)-phenyl-2-methylpropyl]-2,6-dimethyl-morpholinhydrochlorid,

Ethyl-[(4-chlorphenyl)-azo]-cyanoacetat,

Kaliumhydrogencarbonat,

Methantetrathiol-Natriumsalz,

Methyl-1-(2,3-dihydro-2,2-dimethyl-1H-inden-1-yl)-1H-imidazol-5-carboxylat,

Methyl-N-(2,6-dimethylphenyl)-N-(5-isoxazolylcarbonyl)-DL-alaninat,

Methyl-N-(chloracetyl)-N-(2,6-dimethylphenyl)-DL-alaninat,

N-(2,3-Dichlor-4-hydroxyphenyl)-1-methyl-cyclohexancarboxamid.

N-(2,6-Dimethylphenyl)-2-methoxy-N-(tetrahydro-2-oxo-3-furanyl)-acetamid,

N-(2,6-Dimethylphenyl)-2-methoxy-N-(tetrahydro-2-oxo-3-thienyl)-acetamid,

N-(2-Chlor-4-nitrophenyl)-4-methyl-3-nitro-benzolsulfonamid,

N-(4-Cyclohexylphenyl)-1,4,5,6-tetrahydro-2-pyrimidinamin,

N-(4-Hexylphenyl)-1,4,5,6-tetrahydro-2-pyrimidinamin,

N-(5-Chlor-2-methylphenyl)-2-methoxy-N-(2-oxo-3-oxazolidinyl)-acetamid,

N-(6-Methoxy)-3-pyridinyl)-cyclopropancarboxamid,

N-[2,2,2-Trichlor-1-[(chloracetyl)-amino]-ethyl]-benzamid,

N-[3-Chlor-4,5-bis-(2-propinyloxy)-phenyl]-N'-methoxy-methanimidamid,

N-Formyl-N-hydroxy-DL-alanin -Natriumsalz,

O,O-Diethyl-[2-(dipropylamino)-2-oxoethyl]-ethylphosphoramidothioat,

O-Methyl-S-phenyl-phenylpropylphosphoramidothioate,

S-Methyl-1,2,3-benzothiadiazol-7-carbothioat,

spiro[2H]-1-Benzopyran-2,1'(3'H)-isobenzofuran]-3'-on,

**Bakterizide:**

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

**Insektizide / Akarizide / Nematizide:**

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,

Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,

Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,

Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,

Edifenphos, Emamectin, Esfenvalerat, Ethiofencarb, Ethion, Ethoferiprox, Ethoprophos, Etrimphos,

Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,

HCH, Heptenophos, Hexaflumuron, Hexythiazox,

Imidacloprid, Iprobenfos, Isazophos, Isofenphos, Isoprocarb, Isoxathion, Ivermectin,

Lambda-cyhalothrin, Lufenuron,

Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,

Naled, NC 184, NI 25, Nitenpyram

Omethoat, Oxamyl, Oxydemethon M, Oxydeprofos,

Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen,

Quinalphos,

RH 5992,

Salithion, Sebufos, Silafluofen, Sulfotep, Sulprofos, Spinosad,

Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb, Thiamethoxam,

Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide und Nematizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

In entsprechenden Aufwandmengen zeigen die erfindungsgemäßen Verbindungen auch herbizide Eigenschaften bzw. pflanzwachstumsregulierende Wirkung, wie z.B. einen Defoliant-Effekt.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.. Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Omithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Omithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen

(intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpudems sowie mit Hilfe von wirkstoffhaltigen Formkörpern, wie Halsbändern, Ohrmarken, Schwanzmarken, Gliedmaßenbändem, Halftern, Markierungsvorrichtungen usw.

Bei der Anwendung für Vieh, Geflügel, Haustiere etc. kann man die Wirkstoffe der Formel (Ib) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie

Hylotrupes bajulus, Chlorophorus pilosis, Anobium punctatum, Xestobium rufovillosum, Ptilinus pecticornis, Dendrobium pertinex, Emobius mollis, Priobium carpini, Lyctus brunneus, Lyctus africanus, Lyctus planicollis, Lyctus linearis, Lyctus pubescens, Trogoxylon aequale, Minthes rugicollis; Xyleborus spec. Tryptodendron spec. Apate monachus, Bostrychus capucins, Heterobostrychus brunneus, Sinoxylon spec. Dinoderus minutus

Hautflügler wie

Sirex juvencus, Urocerus gigas, Urocerus gigas taignus, Urocerus augur

Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze, wie Lepisma saccarina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und- türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlomaphthalin, vorzugsweise α-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der Wo 94/29 268 genannten Insektizide und Fungizide in Frage. Die in diesem Dokument genannten Verbindungen sind ausdrücklicher Bestandteil der vorliegenden Anmeldung.

Ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Die Herstellung und die Verwendung der erfindungsgemäßen Wirkstoffe gehen aus den nachfolgenden Beispielen hervor.

**Herstellungsbeispiele**

Beispiel 1

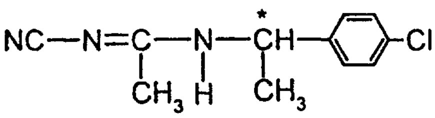

Zu 7,8 g (0,05 Mol) [R]-4-Chlor-2-methylbenzylamin in 30 ml Methanol gibt man 5,0 g (0,05 Mol) N-Cyano-ethanimidsäuremethylester und läßt das Reaktionsgemisch 2 Stunden bei Raumtemperatur rühren. Anschließend wird mit Wasser versetzt, der ausgefallene Niederschlag abfiltriert und getrocknet.
Man erhält 7,0 g (63% der Theorie) [R]-N-Cyano-N'-(4-chlorphenyl-eth-1-yl)-ethanimidamid vom Schmelzpunkt 125°C und einem Drehwinkel $[a]_D^{20}$ = + 252,5° ($CH_3OH$).
Analog bzw. gemäß den allgemeinen Verfahrensangaben werden die folgenden erfindungsgemäßen Verbindungen erhalten:

Tabelle A

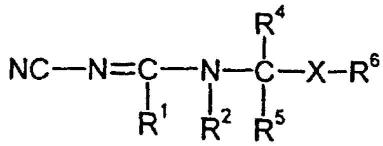

(I b)

| Bsp.-Nr. | $R^1$ | $R^2$ | $R^4$ | $R^5$ | X | $R^6$ | Physik. Konst. |
|---|---|---|---|---|---|---|---|
| 2 | H | H | $CH_3$ | H | - | 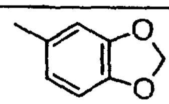 | viskos |
| 3 | $CH_3$ | H | $C_2H_5$ | H | - | 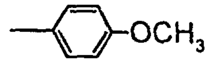 | Fp. 134°C |
| 4 | $CH_3$ | H | H | $CH_3$ | $-CH_2CH_2-$ | 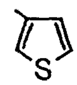 | Fp. 72°C |
| 5 | $CH_3$ | H | H | $C_2H_5$ | $-CH_2CH_2-$ | 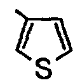 | viskos |
| 6 | $CH_3$ | H | H | $CH_3$ | $-CH_2CH_2-$ | 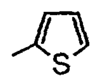 | Fp. 92°C |
| 7 | $CH_3$ | H | H | $C_2H_5$ | $-CH_2CH_2-$ | 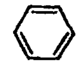 | Fp. 90°C |
| 8 | $CH_3$ | H | H | $CH_3$ | $-CH_2CH_2-$ | 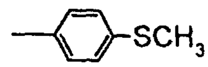 | Fp. 154°C |
| 9 | $CH_3$ | H | H | $CH_3$ | $-CH_2CH_2-$ | 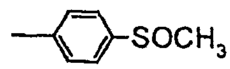 | Öl |
| 10 | $CH_3$ | H | H | $CH_3$ | $-CH_2CH_2-$ | 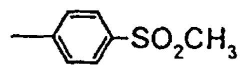 | Fp. 74°C |
| 11 | $CH_3$ | H | H | $n-C_3H_7$ | $-CH_2CH_2-$ | 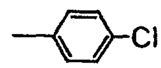 | $n_D^{20} = 1.5476$ |
| 12 | $CH_3$ | H | H | $i-C_3H_7$ | $-CH_2CH_2-$ | 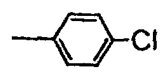 | Fp. 142°C |
| 13 | $CH_3$ | H | H | $C_2H_5$ | $-CH_2CH_2-$ | 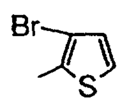 | $n_D^{20} = 1.5760$ |
| 14 | $CH_3$ | H | H | $CH_3$ | $-CH_2CH_2-$ | 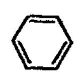 | Fp. 124°C (R-Isomer) |
| 15 | $CH_3$ | H | H | $CH_3$ | $-CH_2CH_2-$ | 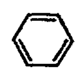 | Fp. 121°C (S-Isomer) |

Tabelle A (Fortsetzung)

| Bsp.-Nr. | $R^1$ | $R^2$ | $R^4$ | $R^5$ | X | $R^6$ | Physik. Konst. |
|---|---|---|---|---|---|---|---|
| 16 | $CH_3$ | H | H | $C_2H_5$ | $-CH_2CH_2-$ | 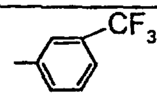 | viskos |
| 17 | $CH_3$ | H | H | $CH_3$ | - | 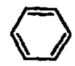 | Fp. 137°C (S-Isomer) |
| 18 | $CH_3$ | H | H | $CH_3$ | $-CH_2CH_2-$ | 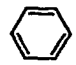 | Fp. 112°C (R-Isomer) |
| 19 | $CH_3$ | H | H | $CH_3$ | $-CH_2CH_2-$ | 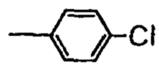 | Fp. 140°C (S-Isomer) |
| 20 | $CH_3$ | H | H | $C_2H_5$ | $-CH_2CH_2-$ | 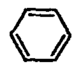 | Fp. 127°C |
| 21 | $CH_3$ | H | H | $C_2H_5$ | $-CH_2CH_2-$ | 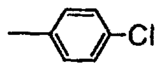 | viskos |
| 22 | $CH_3$ | H | $CH_3$ | $CH_3$ | - | 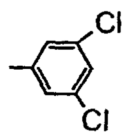 | Fp. 135°C |
| 23 | $CH_3$ | H | H | $CH_3$ | - | 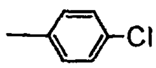 | Fp. 135°C |
| 24 | $CH_3$ | H | H | $C_2H_5$ | - | 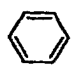 | Fp. 108°C (S-Isomer) |
| 25 | $CH_3$ | H | H | $C_2H_5$ | - | 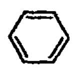 | Fp. 117°C (R-Isomer) |
| 26 | $CH_3$ | H | H | $i-C_3H_7$ | - | 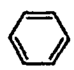 | $n_D^{20} = 1.5517$ |
| 27 | $CH_3$ | H | H | $n-C_3H_7$ | - | 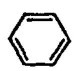 | $n_D^{20} = 1.5522$ |
| 28 | $CH_3$ | H | H | $CH_3$ | - | 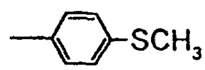 | Fp. 90°C |
| 29 | $CH_3$ | H | H | $CH_3$ | - | 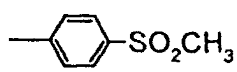 | Fp. 130°C |
| 30 | $CH_3$ | H | H | $CH_3$ | - | 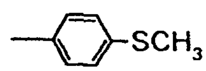 | Fp. 103°C (S-Isomer) |
| 31 | $CH_3$ | H | H | $CH_3$ | - | 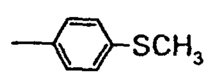 | Fp. 130°C (R-Isomer) |
| 32 | $CH_3$ | H | H | $CH_3$ | - | 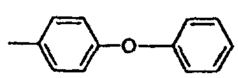 | $n_D^{20} = 1.5861$ |

Tabelle A (Fortsetzung)

| Bsp.-Nr. | $R^1$ | $R^2$ | $R^4$ | $R^5$ | X | $R^6$ | Physik. Konst. |
|---|---|---|---|---|---|---|---|
| 33 | $CH_3$ | H | H | $CH_3$ | - | 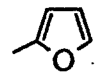 | viskos |
| 34 | $CH_3$ | H | H | $CH_3$ | - | 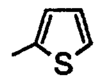 | Öl |
| 35 | $CH_3$ | H | H | $CH_3$ | - | 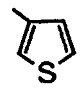 | $n_D^{20}$ = 1.5572 |
| 36 | $CH_3$ | H | H | $C_2H_5$ | - | 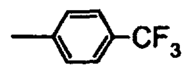 | viskos |
| 37 | $CH_3$ | H | H | $CH_3$ | - | 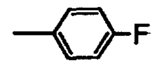 | Fp. 68°C |
| 38 | $CH_3$ | H | H | $C_2H_5$ | - | 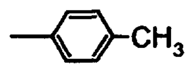 | viskos |
| 39 | $CH_3$ | H | H | $CH_3$ | - | 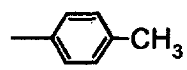 | viskos |
| 40 | $CH_3$ | H | H | $CH_3$ | - | 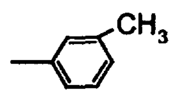 | Fp. 72°C |
| 41 | $CH_3$ | H | H | $C_2H_5$ | - | 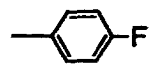 | viskos |
| 41 | $CH_3$ | H | H | $C_2H_5$ | $-CH_2O-$ | 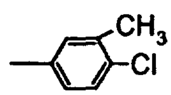 | viskos |
| 43 | $CH_3$ | H | H | $CH_3$ | - | 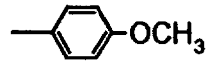 | Fp. 108-10°C |
| 44 | $CH_3$ | H | H | $CH_3$ | - | 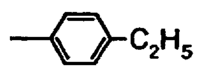 | Öl |
| 45 | $CH_3$ | H | H | $CH_3$ | - | 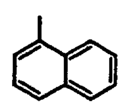 | Fp. 95°C |
| 46 | $CH_3$ | H | H | $CH_3$ | - | 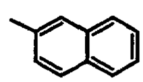 | Fp. 134°C |
| 47 | $CH_3$ | H | H | $C_2H_5$ | - | 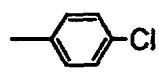 | Fp. 117°C (R-Isomer) |
| 48 | $CH_3$ | H | H | $CH_3$ | - | 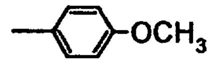 | Fp. 87°C (R-Isomer) |

| | | | | | | | |
|---|---|---|---|---|---|---|---|
| 49 | $CH_3$ | H | H | $C_2H_5$ | - | 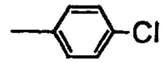 | Fp. 126°C (S-Isomer) |
| 50 | $CH_3$ | H | H | $CH_3$ | - | 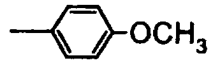 | Fp. 71°C (S-Isomer) |
| 51 | $CH_3$ | H | H | $CH_3$ | $-CH_2CH_2-$ | 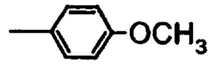 | Fp. 87°C |
| 52 | $CH_3$ | H | H | $CH_3$ | $-CH_2CH_2-$ | 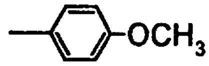 | Fp. 75°C (R-Isomer) |
| 53 | $CH_3$ | H | H | $C_2H_5$ | $-CH_2OCH_2-$ | 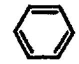 | Öl |
| 54 | $CH_3$ | H | H | $C_2H_5$ | $-CH_2OCH_2-$ | 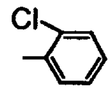 | Fp. 83°C |
| 55 | $CH_3$ | H | H | $C_2H_5$ | $-CH_2OCH_2-$ | 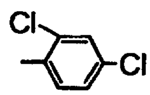 | Fp. 95°C |
| 56 | $CH_3$ | H | H | $C_2H_5$ | $-CH_2OCH_2-$ | 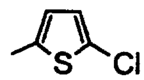 | Öl |
| 57 | $CH_3$ | H | H | $C_2H_5$ | $-CH_2OCH_2-$ | 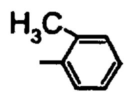 | Fp. 86°C |
| 58 | $CH_3$ | H | H | $C_2H_5$ | $-CH_2OCH_2-$ | 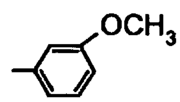 | Öl |
| 59 | $CH_3$ | H | H | $C_2H_5$ | $-CH_2OCH_2-$ | 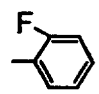 | Öl |
| 60 | $CH_3$ | H | H | $C_2H_5$ | $-CH_2OCH_2-$ | 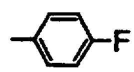 | Öl |
| 61 | $CH_3$ | H | H | $C_2H_5$ | $-CH_2OCH_2-$ | 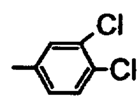 | Öl |
| 62 | $CH_3$ | H | H | $C_2H_5$ | $-CH_2OCH_2-$ | 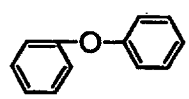 | Öl |
| 63 | $CH_3$ | H | H | $C_2H_5$ | $-CH_2OCH_2-$ | 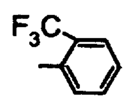 | viskos |
| 64 | $CH_3$ | H | H | $C_2H_5$ | $-CH_2OCH_2-$ | 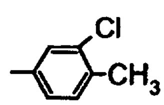 | Fp. 79°C |
| 65 | $CH_3$ | H | H | H | $-CH_2SCH_2-$ | 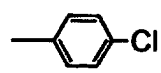 | viskos |

| | | | | | | | |
|---|---|---|---|---|---|---|---|
| 66 | $CH_3$ | $CH_3$ | H | H | - | 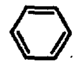 | Öl |
| 67 | $CH_3$ | H | H | H | - | 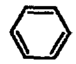 | Fp. 121°C |
| 68 | $CH_3$ | H | H | $C_2H_5$ | $-CH_2S-$ | 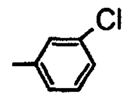 | Fp. 99-102°C |
| 69 | $CH_3$ | H | H | $CH_3$ | $-CH_2S-$ | 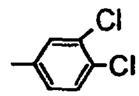 | Öl |
| 70 | $CH_3$ | H | H | $CH_3$ | $-CH_2S-$ | 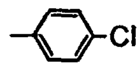 | Öl |
| 71 | $CH_3$ | H | H | $C_2H_5$ | $-CH_2S-$ | 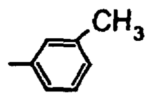 | Öl |
| 72 | $CH_3$ | H | H | $C_2H_5$ | $-CH_2S-$ | 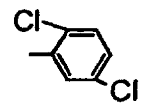 | Öl |
| 73 | $CH_3$ | H | H | $C_2H_5$ | $-CH_2SCH_2-$ | 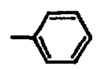 | Öl |
| 74 | $CH_3$ | H | H | $C_2H_5$ | $-CH_2S-$ | 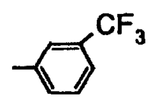 | Öl |
| 75 | $CH_3$ | H | H | $CH_3$ | $-CH_2S-$ | 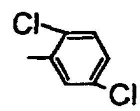 | Fp. 145°C |
| 76 | $CH_3$ | H | H | $C_2H_5$ | $-CH_2S-$ | 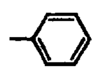 | Öl |
| 77 | $CH_3$ | H | H | $C_2H_5$ | $-CH_2S-$ | 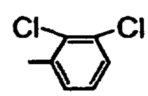 | Fp. 135-38°C |
| 78 | $CH_3$ | H | H | $C_2H_5$ | $-CH_2S-$ | 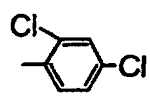 | Fp. 240-43°C |

| | | | | | | | |
|---|---|---|---|---|---|---|---|
| 79 | $CH_3$ | H | H | $C_2H_5$ | $-CH_2S-$ | 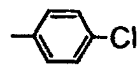 | Öl |
| 80 | $CH_3$ | H | H | $CH_3$ | - | 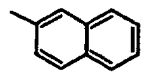 | Fp. 150°C (S-Isomer) |
| 81 | $CH_3$ | H | H | $CH_3$ | - | 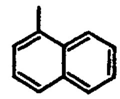 | Fp. 188°C (S-Isomer) |
| 82 | $CH_3$ | H | H | $CH_3$ | $-CH_2CH_2-$ | 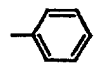 | Fp. 126°C (S-Isomer) |
| 83 | $CH_3$ | H | H | $CH_3$ | - | 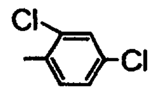 | Fp. 152°C |
| 84 | $CH_3$ | H | H | $C_2H_5$ | $-CH_2O-$ | 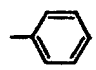 | Fp. 114°C |
| 85 | $CH_3$ | H | H | $C_2H_5$ | $-CH_2O-$ | 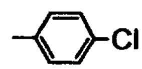 | Fp. 118°C |
| 86 | $CH_3$ | H | H | $C_2H_5$ | $-CH_2O-$ | 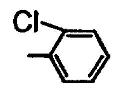 | Fp. 110°C |
| 87 | $CH_3$ | H | H | $C_2H_5$ | $-CH_2O-$ | 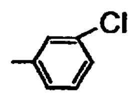 | Fp. 104°C |
| 88 | $CH_3$ | H | H | $C_2H_5$ | $-CH_2O-$ | 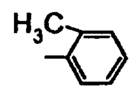 | Fp. 125°C |
| 89 | $CH_3$ | H | H | $C_2H_5$ | $-CH_2O-$ | 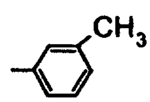 | Fp. 118°C |
| 90 | $CH_3$ | H | H | $C_2H_5$ | $-CH_2O-$ | 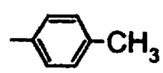 | viskos |
| 91 | $CH_3$ | H | H | $C_2H_5$ | $-CH_2O-$ | 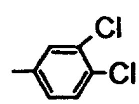 | Fp. 125°C |
| 92 | $CH_3$ | H | H | $C_2H_5$ | $-CH_2O-$ | 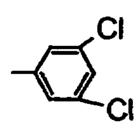 | Fp. 114°C |

Tabelle A (Fortsetzung)

| Bsp.-Nr. | $R^1$ | $R^2$ | $R^4$ | $R^5$ | X | $R^6$ | Physik. Konst. |
|---|---|---|---|---|---|---|---|
| 93 | $CH_3$ | H | H | H | - | 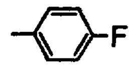 | Fp. 168°C |
| 94 | $CH_3$ | H | H | H | - | 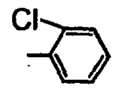 | Fp. 150°C |
| 95 | $CH_3$ | H | H | H | - | 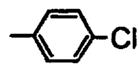 | Fp. 159°C |
| 96 | $CH_3$ | H | H | H | - | 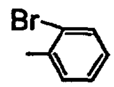 | Fp. 145°C |
| 97 | $CH_3$ | H | H | H | - | 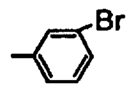 | Fp. 145°C |
| 98 | $CH_3$ | H | H | H | - | 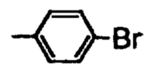 | Fp. 167°C |
| 99 | $CH_3$ | $CH_3$ | H | H | - | 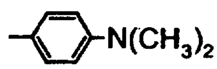 | viskos |
| 100 | $CH_3$ | H | H | H | - | 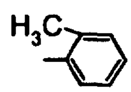 | Fp. 151°C |
| 101 | $CH_3$ | H | H | H | - | 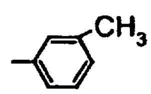 | Fp. 113°C |
| 102 | $CH_3$ | H | H | H | - | 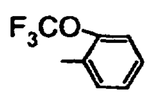 | Fp. 106°C |
| 103 | $CH_3$ | H | H | H | - | 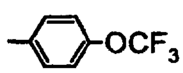 | Fp. 114°C |
| 104 | $CH_3$ | H | H | H | - | 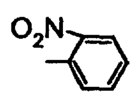 | Fp. 192°C |
| 105 | $CH_3$ | $CH_3$ | H | H | - | 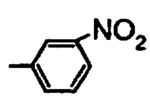 | Fp. 124°C |
| 106 | $CH_3$ | H | H | H | - | 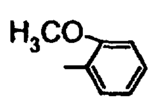 | Fp. 125°C |
| 107 | $CH_3$ | H | H | H | - | 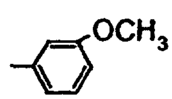 | Fp. 92°C |
| 108 | $CH_3$ | H | H | H | - | 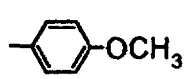 | Fp. 112°C |

Tabelle A (Fortsetzung)

| Bsp.-Nr. | $R^1$ | $R^2$ | $R^4$ | $R^5$ | X | $R^6$ | Physik. Konst. |
|---|---|---|---|---|---|---|---|
| 109 | $CH_3$ | H | H | H | - | 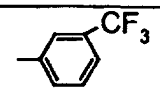 | Fp. 111°C |
| 110 | $CH_3$ | H | H | H | - | 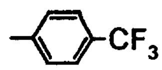 | Fp. 130°C |
| 111 | $CH_3$ | $CH_3$ | H | H | - | 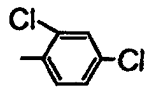 | Fp. 108°C |
| 112 | $CH_3$ | H | H | H | - | 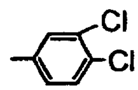 | Fp. 166°C |
| 113 | $CH_3$ | H | H | H | - | 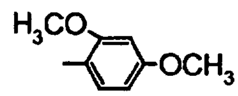 | Fp. 118°C |
| 114 | $CH_3$ | H | H | H | - | 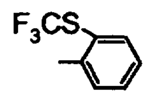 | Fp. 112°C |
| 115 | $CH_3$ | H | H | H | - | 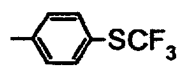 | Fp. 165°C |
| 116 | $CH_3$ | H | H | H | - | 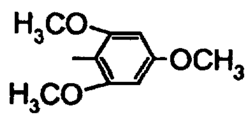 | Fp. 164°C |
| 117 | $CH_3$ | H | H | H | - | 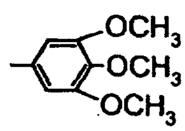 | Fp. 144°C |
| 118 | $CH_3$ | H | H | H | - | 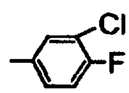 | Fp. 164°C |
| 119 | $CH_3$ | H | H | H | - | 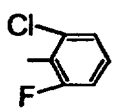 | Fp. 160°C |
| 120 | $CH_3$ | H | H | H | - | 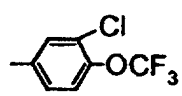 | Fp. 119°C |
| 121 | $CH_3$ | H | H | H | - | 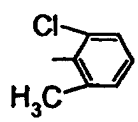 | Fp. 174°C |

Tabelle A (Fortsetzung)

| Bsp.-Nr. | $R^1$ | $R^2$ | $R^4$ | $R^5$ | X | $R^6$ | Physik. Konst. |
|---|---|---|---|---|---|---|---|
| 122 | $CH_3$ | H | H | H | - | 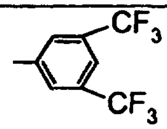 | Fp. 140°C |
| 123 | $CH_3$ | H | H | H | - | 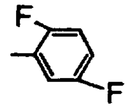 | Fp. 148°C |
| 124 | $CH_3$ | H | H | H | - | 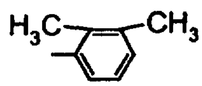 | Fp. 147°C |
| 125 | $CH_3$ | H | H | H | - | 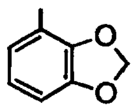 | Fp. 154°C |
| 126 | $CH_3$ | H | H | H | - | 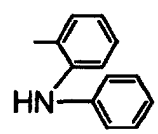 | Fp. 134°C |
| 127 | $CH_3$ | H | H | $C_3H_7$-i | - | 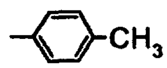 | Fp. 138°C |
| 128 | $CH_3$ | H | $CH_3$ | $CH_3$ | -C≡C- | 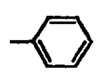 | Fp. 117°C |
| 129 | $CH_3$ | H | $CH_3$ | $CH_3$ | -C≡C- | 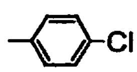 | Fp. 153°C |
| 130 | $CH_3$ | H | $CH_3$ | $CH_3$ | -C≡C- | 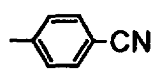 | viskos |
| 131 | $CH_3$ | H | $CH_3$ | $CH_3$ | -C≡C- | 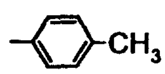 | Fp. 138°C |
| 132 | $CH_3$ | H | $CH_3$ | $CH_3$ | -C≡C- | 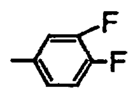 | Fp. 122°C |
| 133 | $CH_3$ | H | $CH_3$ | H | - | 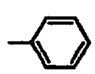 | Fp. 90°C |
| 134 | $CH_3$ | H | $CH_3$ | H | - | 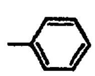 | Fp. 110°C (R-Isomer) |
| 135 | $CH_3$ | H | $CH_3$ | H | - | 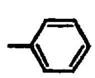 | Fp. 134°C (S-Isomer) |

Tabelle A (Fortsetzung)

| Bsp.-Nr. | $R^1$ | $R^2$ | $R^4$ | $R^5$ | X | $R^6$ | Physik. Konst. |
|---|---|---|---|---|---|---|---|
| 136 | $CH_3$ | H | $CH_3$ | H | - | 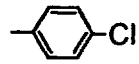 | viskos |
| 137 | $CH_3$ | H | $CH_3$ | H | - | 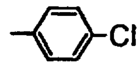 | Fp. 131°C (R-Isomer) |
| 138 | $CH_3$ | H | $CH_3$ | H | - | 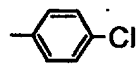 | viskos (S-Isomer) |
| 139 | $CH_3$ | H | $C_2H_5$ | H | - | 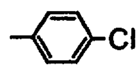 | viskos |
| 140 | $CH_3$ | H | $C_3H_7$-n | H | - | 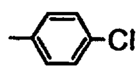 | Fp. 86°C |
| 141 | $CH_3$ | H | $CH_3$ | H | - | 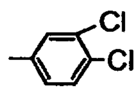 | Fp. 156°C |
| 142 | $CH_3$ | H | $CH_3$ | H | - | 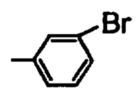 | Fp. 131°C |
| 143 | $CH_3$ | H | $CH_3$ | H | - | 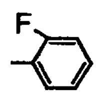 | Fp. >250°C |
| 144 | $CH_3$ | H | $CH_3$ | H | - | 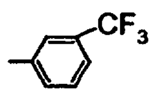 | Fp. 105°C |
| 145 | $CH_3$ | H | $CH_3$ | H | - | 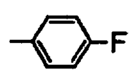 | Fp. 115°C |
| 146 | $CH_3$ | H | $CH_3$ | H | - | 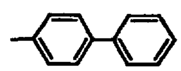 | Fp. 110°C |
| 147 | $CH_3$ | H | $CH_3$ | H | - | 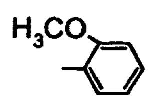 | Fp. 135°C |
| 148 | $CH_3$ | H | $CH_3$ | H | - | 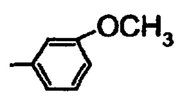 | Fp. 140°C |
| 149 | $CH_3$ | H | $CH_3$ | H | $-CH_2CH_2-$ | 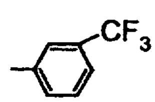 | Fp. 95°C |
| 150 | $CH_3$ | H | $CH_3$ | H | $-CH_2CH_2-$ | 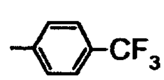 | viskos |
| 151 | $CH_3$ | H | $CH_3$ | H | $-CH_2CH_2-$ | 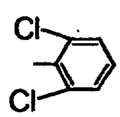 | viskos |

Tabelle A (Fortsetzung)

| Bsp.-Nr. | $R^1$ | $R^2$ | $R^4$ | $R^5$ | X | $R^6$ | Physik. Konst. |
|---|---|---|---|---|---|---|---|
| 152 | $CH_3$ | H | H | H | $-CH(CH_3)-$ | 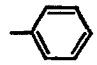 | Fp. 96°C |
| 153 | $CH_3$ | H | $CH_3$ | H | $-CH(CH_3)CH_2-$ | 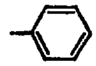 | viskos |
| 154 | $CH_3$ | H | $CH_3$ | H | $-CH(CH_3)CH_2-$ | 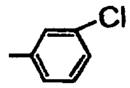 | viskos |
| 155 | $CH_3$ | H | $CH_3$ | H | $-CH(CH_3)CH_2-$ | 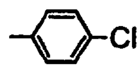 | Fp. 108°C |
| 156 | $CH_3$ | H | $CH_3$ | H | $-CH(CH_3)CH_2-$ | 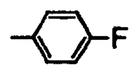 | viskos |
| 157 | $CH_3$ | H | $CH_3$ | H | $-CH(CH_3)CH_2-$ | 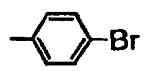 | Fp. 122°C |
| 158 | $CH_3$ | H | $CH_3$ | H | $-CH(CH_3)CH_2-$ | 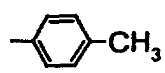 | viskos |
| 159 | $CH_3$ | H | $CH_3$ | H | $-CH(CH_3)CH_2-$ | 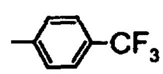 | Fp. 120°C |
| 160 | $CH_3$ | H | $CH_3$ | H | - | 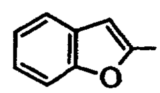 | Fp. 134°C |
| 161 | $CH_3$ | H | $CH_3$ | H | - | 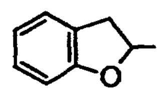 | viskos |
| 162 | $CH_3$ | H | H | H | $-C(CH_3)=CH-$ | 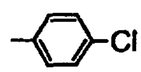 | Fp. 79°C |
| 163 | $CH_3$ | H | H | H | $-C(CH_3)_2-$ | 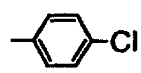 | Fp. 169°C |
| 164 | $CH_3$ | H | $CH_3$ | H | $-CH_2C(CH_3)_2-$ | 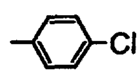 | viskos |
| 165 | $CH_3$ | H | $CH_3$ | H | - | 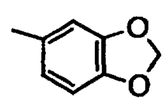 | viskos |
| 166 | $CH_3$ | H | $C_2H_5$ | H | $-CH_2-$ | 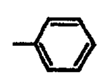 | viskos |
| 167 | $CH_3$ | H | $CH_3$ | H | $-CH_2CH_2-$ | 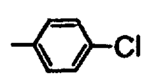 | viskos |
| 168 | $CH_3$ | H | $CH_3$ | H | $-CH_2-$ | 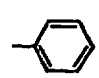 | Fp. 84°C |

Tabelle A (Fortsetzung)

| Bsp.-Nr. | $R^1$ | $R^2$ | $R^4$ | $R^5$ | X | $R^6$ | Physik. Konst. |
|---|---|---|---|---|---|---|---|
| 169 | $CH_3$ | H | H | H | $-CH(CH_3)CH_2-$ | 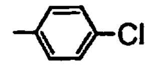 | viskos |
| 170 | $CH_3$ | H | H | H | $-CH(CH_3)CH_2-$ | 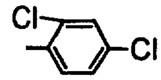 | viskos |
| 171 | $CH_3$ | H | $CH_3$ | H | $-CH_2O-$ | 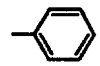 | viskos |
| 172 | $CH_3$ | H | $CH_3$ | H | $-CH_2O-$ | 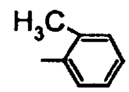 | viskos |
| 173 | $CH_3$ | H | $CH_3$ | H | $-CH_2O-$ | 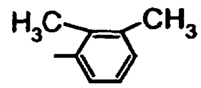 | Fp. 118°C |
| 174 | $CH_3$ | H | $CH_3$ | H | $-CH_2O-$ | 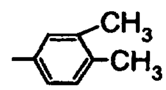 | viskos |
| 175 | $CH_3$ | H | $CH_3$ | H | $-(CH_2)_3-N(C_2H_5)-CH_2-$ | 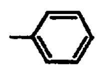 | viskos |
| 176 | H | H | $CH_3$ | $CH_3$ | $-CH_2CH_2-$ | 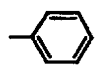 | Fp. 74°C |
| 177 | H | H | $CH_3$ | H | $-CH_2CH_2-$ | 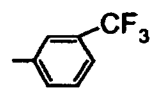 | Fp. 76°C |
| 178 | H | H | $CH_3$ | H | $-CH_2CH_2-$ | 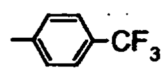 | viskos |
| 179 | H | H | $CH_3$ | H | $-CH_2CH_2-$ | 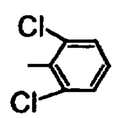 | viskos |
| 180 | H | H | H | H | $-CH(CH_3)-$ | 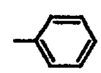 | viskos |
| 181 | H | H | $CH_3$ | H | $-CH(CH_3)CH_2-$ | 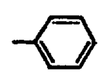 | viskos |
| 182 | H | H | $CH_3$ | $CH_3$ | $-C\equiv C-$ | 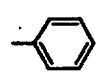 | Fp. 75°C |
| 183 | H | H | $CH_3$ | $CH_3$ | $-C\equiv C-$ | 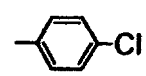 | Fp. 114°C |
| 184 | H | H | $CH_3$ | $CH_3$ | $-C\equiv C-$ | 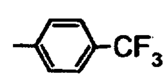 | Fp. 80°C |
| 185 | H | H | $CH_3$ | $CH_3$ | $-C\equiv C-$ | 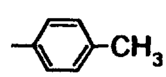 | Fp. 100°C |

Tabelle A (Fortsetzung)

| Bsp.-Nr. | $R^1$ | $R^2$ | $R^4$ | $R^5$ | X | $R^6$ | Physik. Konst. |
|---|---|---|---|---|---|---|---|
| 186 | H | H | $CH_3$ | $CH_3$ | -C≡C- | 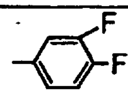 | viskos |
| 187 | H | H | $CH_3$ | $CH_3$ | -C≡C- | 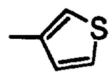 | Fp. 104°C |
| 188 | H | H | $CH_3$ | H | - | 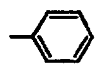 | viskos |
| 189 | H | H | $CH_3$ | H | - | 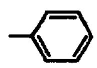 | Fp. 66°C (R-Isomer) |
| 190 | H | H | $CH_3$ | H | - | 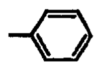 | Fp. 68°C (S-Isomer) |
| 191 | H | H | $CH_3$ | H | - | 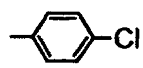 | viskos |
| 192 | H | H | $C_2H_5$ | H | - | 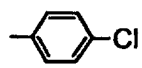 | viskos |
| 193 | H | H | $C_3H_7$-n | H | - | 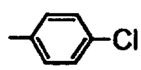 | viskos |
| 194 | H | H | $CH_3$ | H | $-CH_2CH_2-$ | 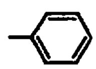 | viskos |
| 195 | H | H | H | H | $-CH(CH_3)CH_2-$ | 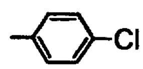 | Fp. 95°C |
| 196 | H | H | $CH_3$ | H | - | 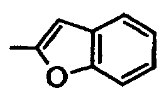 | Fp. 78°C |
| 197 | H | H | H | H | $-C(CH_3)_2-$ | 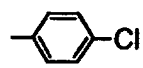 | Fp. 84°C |
| 198 | H | H | $CH_3$ | H | $-CH_2-C(CH_3)_2-$ | 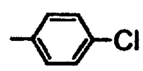 | viskos |
| 199 | $CH_3$ | H | H | H | $-CH_2-$ | 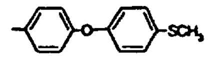 | Fp. 153°C |
| 200 | $CH_3$ | $CH_3$ | H | $CH_3$ | - | 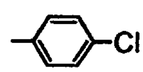 | log P (pH2.3) 2.32 |
| 201 | $CH_3$ | H | H | $CH_3$ | | 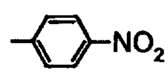 | log P (pH2.3) 1.63 |
| 202 | $CH_3$ | H | H | $C_2H_5$ | - | 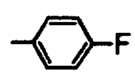 | Fp. 143°C (S-Isomer) |

Tabelle A (Fortsetzung)

| Bsp.-Nr. | $R^1$ | $R^2$ | $R^4$ | $R^5$ | X | $R^6$ | Physik. Konst. |
|---|---|---|---|---|---|---|---|
| 203 | $CH_3$ | H | H | $C_2H_5$ | - | 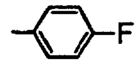 | Fp. 135°C (R-Isomer) |
| 204 | $CH_3$ | H | H | $C_2H_5$ | $-CH_2-CH_2-$ | 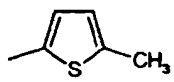 | Wachs |
| 205 | $CH_3$ | H | H | $CH_3$ | - | 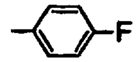 | log P(pH2.3)1.71 (R-Isomer) |
| 206 | $C_3H_7$-n | H | H | $CH_3$ | - | 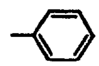 | Öl |
| 207 | $C_3H_7$-n | H | H | $CH_3$ | - | 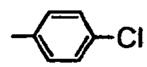 | Fp. 135°C |
| 208 | H | H | H | $CH_3$ | - | 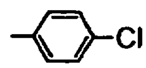 | Öl (R-Isomer) |
| 209 | H | H | H | $CH_3$ | - | 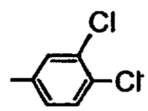 | Öl |
| 210 | H | H | H | H | $-CH_2CH_2-$ | 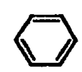 | Öl |
| 211 | H | H | H | $CH_3$ | - | 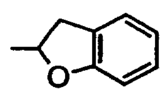 | Öl |
| 212 | H | H | H | $CH_3$ | $-CH_2C(CH_3)_2-$ | 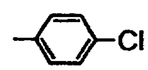 | Öl |
| 213 | H | H | H | $CH_3$ | $-CH_2CH_2-$ | 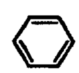 | Öl |
| 214 | H | H | H | $CH_3$ | $-CH(CH_3)CH_2$ | 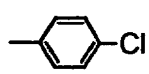 | Öl |
| 215 | H | H | $CH_3$ | $CH_3$ | $-C\equiv C-$ | 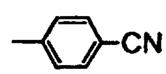 | Öl |
| 216 | H | H | H | $CH_3$ | - | 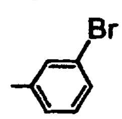 | Öl |

Tabelle A (Fortsetzung)

| Bsp.-Nr. | $R^1$ | $R^2$ | $R^4$ | $R^5$ | X | $R^6$ | Physik. Konst. |
|---|---|---|---|---|---|---|---|
| 217 | H | H | H | $CH_3$ | - | 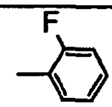 | Öl |
| 218 | H | H | H | $CH_3$ | - | 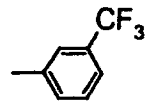 | Öl |
| 219 | H | H | H | $CH_3$ | - | 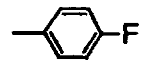 | Öl |
| 220 | H | H | H | $CH_3$ | - | 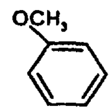 | Öl |
| 221 | H | H | H | $CH_3$ | - | 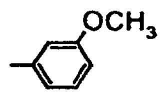 | Öl |
| 222 | $C_2H_5$ | H | H | $CH_3$ | - | 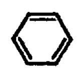 | Öl |
| 223 | $C_2H_5$ | H | H | $CH_3$ | - | 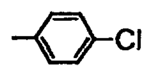 | Fp. 111°C |
| 224 | $C_2H_5$ | H | H | $C_2H_5$ | - | 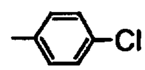 | Öl |
| 225 | $C_2H_5$ | H | H | $C_3H_7$-n | - | 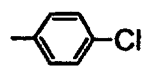 | Öl |
| 226 | $C_2H_5$ | H | H | $CH_3$ | - | 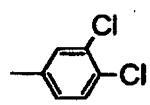 | Fp. 109°C |
| 227 | $C_2H_5$ | H | H | $CH_3$ | - | 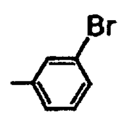 | Öl |
| 228 | $C_2H_5$ | H | H | $CH_3$ | - | 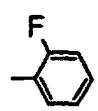 | Fp. 87°C |
| 229 | $C_2H_5$ | H | H | $CH_3$ | - | 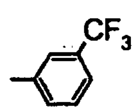 | Fp. 81°C |
| 230 | $C_2H_5$ | H | H | $CH_3$ | - | 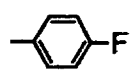 | Öl |
| 231 | $C_2H_5$ | H | H | $CH_3$ | - | 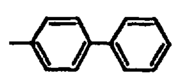 | Fp. 163°C |
| 232 | $C_2H_5$ | H | H | $CH_3$ | - | 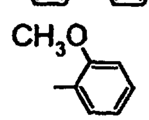 | Fp. 115°C |

Tabelle A (Fortsetzung)

| Bsp.-Nr. | $R^1$ | $R^2$ | $R^4$ | $R^5$ | X | $R^6$ | Physik. Konst. |
|---|---|---|---|---|---|---|---|
| 233 | $C_2H_5$ | H | H | $CH_3$ | - | 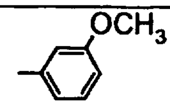 | Öl |
| 234 | $C_2H_5$ | H | H | $CH_3$ | - | 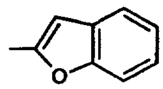 | Öl |
| 235 | $CH_3$ | H | H | H | $-CH_2-$ | 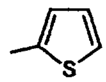 | Fp. 107°C |
| 236 | $CH_3$ | H | H | H | - | 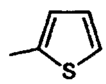 | F. 113°C |
| 237 | $CH_3$ | H | H | H | - | 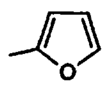 | Fp. 101°C |
| 238 | $CH_3$ | H | H | H | $-CH_2-$ | 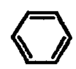 | Fp. 138°C |
| 239 | $CH_3$ | H | H | H | $-CH_2CH_2-$ | 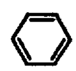 | Fp. 67°C |
| 240 | $CH_3$ | H | H | H | - | 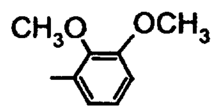 | Öl |
| 241 | $CH_3$ | H | H | H | - | 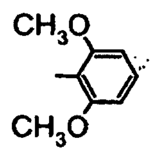 | Fp. 86°C |
| 242 | $CH_3$ | H | H | H | - | 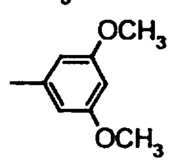 | Fp. 132°C |
| 243 | $CH_3$ | H | H | H | $-CH_2$ | 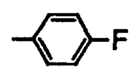 | Fp. 133°C |
| 244 | $CH_3$ | H | H | $CH_3$ | $-CH_2O-$ | 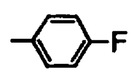 | Öl |
| 245 | H | H | $CH_3$ | $CH_3$ | $-CH_2CH_2-$ | 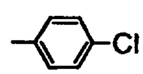 | Fp. 107°C |
| 246 | H | H | H | $CH_3$ | $-CH(CH_3)CH_2-$ | 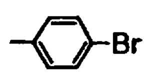 | Öl |
| 247 | H | H | H | $CH_3$ | $-CH(CH_3)CH_2-$ | 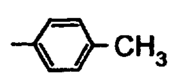 | Öl |
| 248 | H | H | H | H | $-CH(CH_3)CH_2-$ | 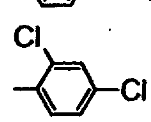 | Öl |

Tabelle A (Fortsetzung)

| Bsp.-Nr. | $R^1$ | $R^2$ | $R^4$ | $R^5$ | X | $R^6$ | Physik. Konst. |
|---|---|---|---|---|---|---|---|
| 249 | H | H | H | $CH_3$ | $-CH_2O-$ | 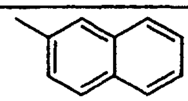 | Fp. 126°C |
| 250 | H | H | H | $CH_3$ | $-CH_2O-$ | 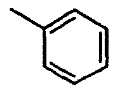 | Öl |
| 251 | H | H | H | $CH_3$ | $-CH_2O-$ | 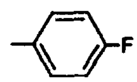 | Öl |
| 252 | H | H | H | $CH_3$ | $-CH_2O-$ | 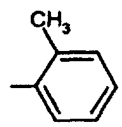 | Öl |
| 253 | H | H | H | $CH_3$ | $-CH_2O-$ | 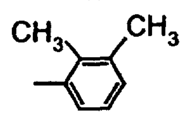 | Fp. 108° C |
| 254 | H | H | H | $CH_3$ | $-CH_2O-$ | 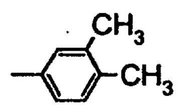 | Fp. 112°C |
| 255 | $C_3H_7$-n | H | H | $CH_3$ | - | 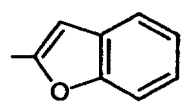 | Öl |
| 256 | H | H | H | H | $-CH_2-$ | 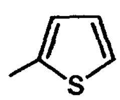 | Öl |
| 257 | H | H | H | H | - | 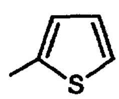 | Öl |
| 258 | H | H | H | H | - | 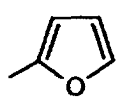 | Öl |
| 259 | H | H | H | H | $-CH_2-$ | 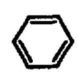 | Öl |
| 260 | H | H | H | H | - | 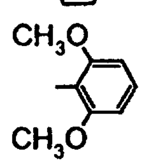 | Fp. 69°C |
| 261 | H | H | H | H | $-CH_2-$ | 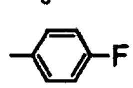 | Öl |
| 262 | H | H | H | H | - | 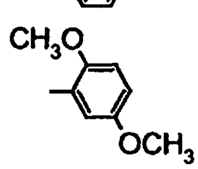 | Fp. 77°C |

Tabelle A (Fortsetzung)

| Bsp.-Nr. | $R^1$ | $R^2$ | $R^4$ | $R^5$ | X | $R^6$ | Physik. Konst. |
|---|---|---|---|---|---|---|---|
| 263 | $CH_3$ | H | H | H | - | 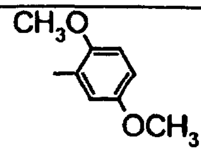 | Fp. 135°C |
| 264 | $CH_3$ | H | H | H | $-(CH_2)_3-$ | 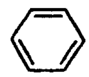 | Fp. 106°C |
| 265 | H | H | H | H | $-(CH_2)_3-$ | 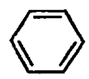 | Öl |
| 266 | $C_2H_5$ | H | H | $CH_3$ | - | 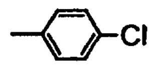 | Fp. 136°C ((+)-Isomer) |
| 267 | $C_2H_5$ | H | H | $CH_3$ | | 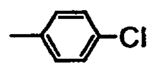 | Fp. 125°C ((-)-Isomer) |
| 268 | $C_3H_7$-n | H | H | $CH_3$ | | 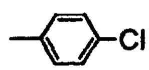 | Fp. 79°C |
| 269 | $C_3H_7$-n | H | H | $CH_3$ | - | 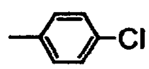 | Fp. 118°C ((+)-Isomer) |
| 270 | $C_3H_7$-n | H | H | $CH_3$ | | 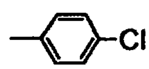 | Fp. 116°C ((-)-Isomer) |
| 271 | $CH_3$ | H | H | $C_2H_5$ | $-CH_2OCH_2$ | 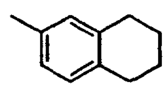 | Öl |
| 272 | $CH_3$ | H | H | $C_2H_5$ | $-CH_2OCH_2$ | 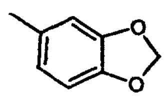 | Öl |
| 273 | $CH_3$ | H | H | $C_2H_5$ | $-CH_2OCH_2-$ | 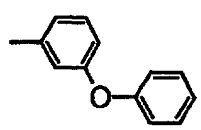 | Öl (R-Isomer) |
| 274 | $CH_3$ | H | H | $C_2H_5$ | - | 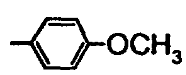 | Fp. 134°C |
| 275 | $CH_3$ | H | H | $C_2H_5$ | | 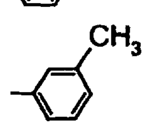 | Fp. 62°C |
| 276 | $CH_3$ | H | H | $C_2H_5$ | - | 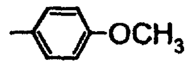 | Wachs (S-Isomer) |

Tabelle A (Fortsetzung)

| Bsp.-Nr. | $R^1$ | $R^2$ | $R^4$ | $R^5$ | X | $R^6$ | Physik. Konst. |
|---|---|---|---|---|---|---|---|
| 277 | $CH_3$ | H | H | $C_2H_5$ | - | 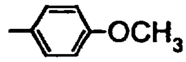 | Fp. 134°C (R-Isomer) |
| 278 | $CH_3$ | H | H | $C_2H_5$ | | 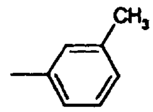 | Fp. 98°C (R-Isomer) |
| 279 | $CH_3$ | H | H | $C_2H_5$ | - | 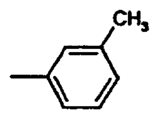 | Fp. 98°C (S-Isomer) |
| 280 | $CH_3$ | H | H | $C_2H_5$ | - | 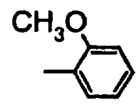 | Fp. 103°C (S-Isomer) |
| 281 | $CH_3$ | H | H | $C_2H_5$ | - | 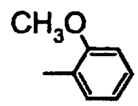 | log P (pH 2,3) 2,05 (R-Isomer) |
| 282 | $CH_3$ | H | H | $CH_3$ | | 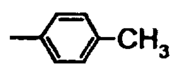 | log P (pH 2,3) 1,93 (S-Isomer) |
| 283 | $CH_3$ | H | H | $CH_3$ | - | 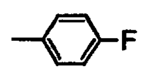 | log P (pH 2,3) 1,71 (S-Isomer) |
| 284 | $CH_3$ | H | H | $CH_3$ | - | 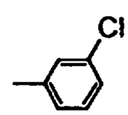 | Fp. 148°C (S-Isomer) |
| 285 | $CH_3$ | H | H | $CH_3$ | | 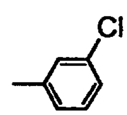 | Fp. 139°C (R-Isomer) |
| 286 | $CH_3$ | H | H | $C_2H_5$ | | 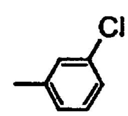 | Fp. 134°C |
| 287 | $CH_3$ | H | H | $C_2H_5$ | | 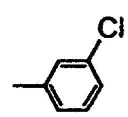 | Fp.113°C (S-Isomer) |
| 288 | $CH_3$ | H | H | $CH_3$ | - | 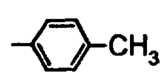 | log P (pH 2,3) 1,94 (R-Isomer) |

Tabelle A (Fortsetzung)

| Bsp.-Nr. | $R^1$ | $R^2$ | $R^4$ | $R^5$ | X | $R^6$ | Physik. Konst. |
|---|---|---|---|---|---|---|---|
| 289 | $CH_3$ | H | H | $C_2H_5$ | - | 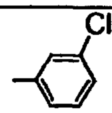 | Fp. 118°C (R-Isomer) |
| 290 | $CH_3$ | H | H | $CH_3$ | - | 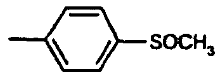 | Wachs |
| 291 | $CH_3$ | H | H | $CH_3$ | - | 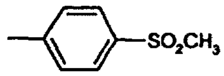 | Fp. 148°C (S-Isomer) |
| 292 | $CH_3$ | H | H | $CH_3$ | - | 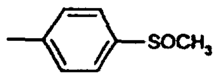 | log P (pH 2,3) 0,78 (R-Isomer) |
| 293 | $CH_3$ | H | H | $CH_3$ | - | 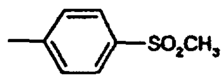 | Fp. 149°C (R-Isomer) |
| 294 | $CH_3$ | H | H | $CH_3$ | - | 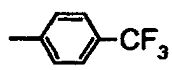 | Fp. 140°C |
| 295 | $CH_3$ | H | H | $CH_3$ | - | 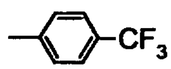 | Fp.138°C (S-Isomer) |
| 296 | $CH_3$ | H | H | $CH_3$ | - | 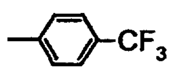 | Fp. 133°C (R-Isomer) |
| 297 | $CH_3$ | H | H | $CH_3$ | - | 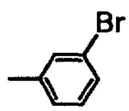 | Fp. 141°C |
| 298 | $CH_3$ | H | H | $CH_3$ | - | 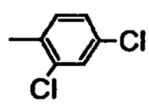 | Fp. 193°C |
| 299 | $CH_3$ | H | H | $CH_3$ | - | 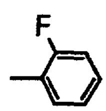 | Fp.132°C |
| 300 | $CH_3$ | H | H | $CH_3$ | - | 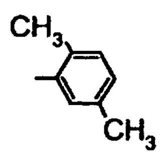 | Fp. 158°C |

Tabelle A (Fortsetzung)

| Bsp.-Nr. | $R^1$ | $R^2$ | $R^4$ | $R^5$ | X | $R^6$ | Physik. Konst. |
|---|---|---|---|---|---|---|---|
| 301 | $CH_3$ | H | H | $C_3H_7$-i | - | 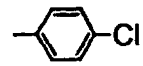 | log P (pH2,3) 2,58 |
| 302 | $CH_3$ | H | H | $CH_3$ | - | 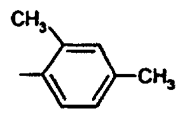 | Fp. 133°C |
| 303 | $CH_3$ | H | H | $CH_3$ | - | 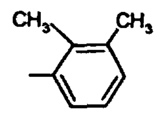 | Fp. 147°C |
| 304 | $CH_3$ | H | H | $CH_3$ | - | 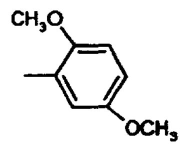 | Fp. 126°C |
| 305 | $CH_3$ | H | H | $C_2H_5$ | $-CH_2CH_2$ | 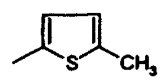 | Wachs |
| 306 | $CH_3$ | H | H | $CH_3$ | - | 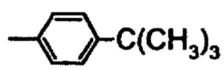 | Fp. 133°C |
| 307 | $CH_3$ | H | H | $CH_3$ | - | 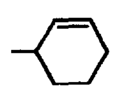 | viskos |
| 308 | $CH_3$ | H | H | $CH_3$ | - | 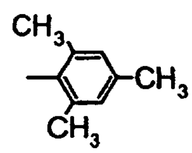 | Fp. 136°C |
| 309 | $CH_3$ | H | H | $CH_3$ | $-CH_2CH_2-$ | 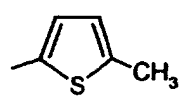 | |
| 310 | $C_2H_5$ | H | H | $CH_3$ | $-CH_2CH_2-$ | 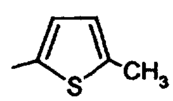 | |
| 311 | $C_3H_7$-n | H | H | $CH_3$ | $-CH_2CH_2-$ | 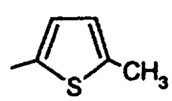 | |
| 312 | $C_3H_7$-i | H | H | $CH_3$ | $-CH_2CH_2-$ | 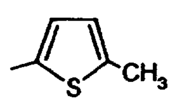 | |

Tabelle A (Fortsetzung)

| Bsp.-Nr. | $R^1$ | $R^2$ | $R^4$ | $R^5$ | X | $R^6$ | Physik. Konst. |
|---|---|---|---|---|---|---|---|
| 313 | $C_2H_5$ | H | H | $C_2H_5$ | $-CH_2CH_2-$ | 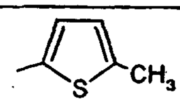 | |
| 314 | $C_3H_7$-n | H | H | $C_2H_5$ | $-CH_2CH_2-$ | 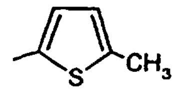 | |
| 315 | $C_3H_7$-i | H | H | $C_2H_5$ | $-CH_2CH_2-$ | 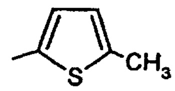 | |

**Anwendungsbeispiele**

Beispiel A

**Grenzkonzentration - Test**

| | |
|---|---|
| Testnematode | **Meloidogyne incognita** |
| Lösungsmittel | 4 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt, der mit den Testnematoden verseucht ist. Dabei spielt die Konzentration des Wirkstoffes in der Zubereitung praktisch keine Rolle, entscheidend ist allein die Wirkstoffmenge pro Volumeneinheit Boden, welche in ppm (= mg/l) angegeben wird. Man füllt den behandelten Boden in Töpfe, sät Salat ein und hält die Töpfe bei einer Gewächshaustemperatur von 25°C.

Nach drei Wochen werden die Salatwurzeln auf Nematodenbefall (Wurzelgallen) untersucht und der Wirkungsgrad des Wirkstoffes in % bestimmt. Der Wirkungsgrad ist 100%, wenn der Befall vollständig vermieden wird, er ist 0%, wenn der Befall genau so hoch ist wie bei den Kontrollpflanzen in unbehandeltem, aber in gleicher Weise verseuchtem Boden.

Bei diesem Test bewirkt z.B. die Verbindung des Herstellungsbeispiels 1 bei einer beispielhaften Wirkstoffkonzentration von 20 ppm eine Abtötung von 100%.

**Patentansprüche**

**1.** Verbindungen der Formel (Ib)

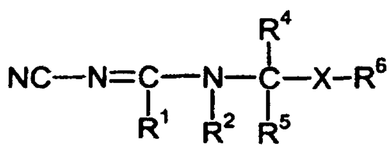

(I b)

in welcher

$R^1$ für Wasserstoff, für gegebenenfalls durch Halogen, Cyano oder $C_1$-$C_4$-Alkoxy substituiertes $C_1$-$C_4$-Alkyl, für gegebenenfalls einfach bis dreifach, gleich oder verschieden durch Halogen oder $C_1$-$C_4$-Alkyl substituiertes $C_3$-$C_6$-Cycloalkyl oder für einfach bis dreifach, gleich oder verschieden substituiertes Phenyl steht, wobei als Substituenten genannt seien:
Hydroxy, Amino, Cyano, Nitro, Halogen; jeweils gegebenenfalls durch Hydroxy, Cyano oder Halogen substituiertes $C_1$-$C_4$-Alkyl, $C_1$-$C_4$-Alkoxy, $C_1$-$C_4$-Alkylamino und Di-($C_1$-$C_4$-)Alkylamino; jeweils gegebenenfalls durch Halogen substituiertes $C_1$-$C_4$-Alkyl-carbonyl, $C_1$-$C_4$-Alkoxy-carbonyl, $C_1$-$C_4$-Alkyl-carbonyl-amino, $C_1$-$C_4$-Alkylthio, $C_1$-$C_4$-Alkylsulfinyl und $C_1$-$C_4$-Alkylsulfonyl; Aminocarbonyl, Aminothiocarbonyl, $C_1$-$C_4$-Alkylaminocarbonyl, Di-($C_1$-$C_4$)-alkylamino-carbonyl, Aminosulfonyl, $C_1$-$C_4$-Alkylaminosulfonyl und Di-($C_1$-$C_4$)-alkylamino-sulfonyl; sowie jeweils gegebenenfalls durch Hydroxy, Cyano, Nitro, Halogen, $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl und $C_1$-$C_4$-Halogenalkyl, $C_1$-$C_4$-Halogenalkoxy, $C_1$-$C_4$-Halogenalkylthio, $C_1$-$C_4$-Halogenalkylsulfinyl und $C_1$-$C_4$-Halogenalkylsulfonyl mit jeweils 1 bis 5 gleichen oder verschiedenen Halogenatomen aus der Reihe Fluor, Chlor und Brom substituiertes Phenyl, Phenoxy, Phenylthio, Phenylsulfinyl, Phenylsulfonyl, Benzyl und Phenylamino,

$R^2$ für Wasserstoff, für gegebenenfalls durch Halogen, Cyano oder $C_1$-$C_4$-Alkoxy substituiertes $C_1$-$C_4$-Alkyl oder für gegebenenfalls einfach bis dreifach, gleich oder verschieden durch Halogen oder $C_1$-$C_4$-Alkyl substituiertes $C_3$-$C_6$-Cycloalkyl steht,

$R^4$ und $R^5$ unabhängig voneinander für Wasserstoff, für gegebenenfalls durch Halogen, Cyano oder $C_1$-$C_4$-Alkoxy substituiertes $C_1$-$C_4$-Alkyl oder für gegebenenfalls einfach bis dreifach, gleich oder verschieden durch Halogen oder $C_1$-$C_4$-Alkyl substituiertes $C_3$-$C_6$-Cycloalkyl stehen,

$R^6$ für gegebenenfalls einfach bis dreifach, gleich oder verschieden substituiertes Phenyl oder Naphthyl steht, wobei als Substituenten genannt seien:
Cyano, Nitro, Amino, Fluor, Chlor, Brom, Methyl, Ethyl, n- oder i-Propyl, n-, i-, s- oder t-Butyl, Methoxy, Ethoxy, n- oder i-Propoxy, Trifluormethyl, Difluormethyl, Trifluormethoxy, Difluormethoxy, Trifluormethylthio, Methylamino, Dimethylamino, Acetyl, Propionyl, Methoxycarbonyl, Ethoxycarbonyl, Methylthio, Methylsulfinyl, Methylsulfonyl, Methylaminocarbonyl, Ethylaminocarbonyl, n- oder i-Propylaminocarbonyl, Dimethylaminocarbonyl und Diethylaminocarbonyl; sowie jeweils gegebenenfalls durch Cyano, Nitro, Fluor, Chlor, Methyl, Methoxy, Methylthio, Methylsulfinyl, Methylsulfonyl, Trifluormethyl oder Trifluormethoxy, Trifluormethylthio, Trifluormethylsulfinyl oder Trifluormethylsulfonyl substituiertes Phenyl, Phenoxy, Phenylthio, Phenylsulfinyl, Phenylsulfonyl, Benzyl oder Phenylamino,
ferner für jeweils gegebenenfalls einfach bis dreifach, gleich oder verschieden durch Fluor, Chlor, Methyl, Ethyl, i-Propyl, Methoxy, Ethoxy, Trifluormethyl oder Trifluormethoxy substituiertes Cyclopentan oder Cyclohexan steht,
sowie für die folgenden, gegebenenfalls einfach bis fünffach, gleich oder verschieden substituierten Heterocyclen steht:

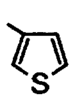 , 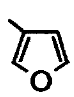 , 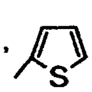 , 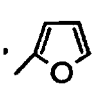 , 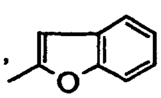 , 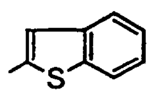 , 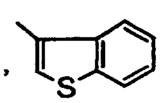 ,

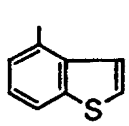 , 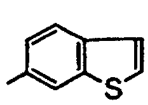 , 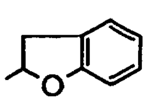 , 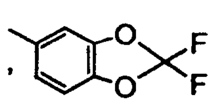 , 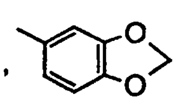 ,

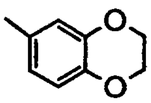, 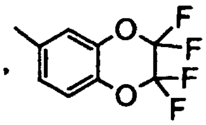, 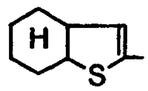, 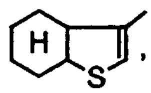, 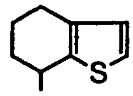,

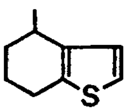, 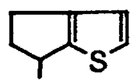, 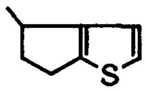, 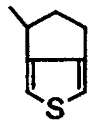, 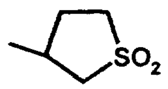, 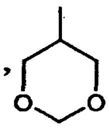,

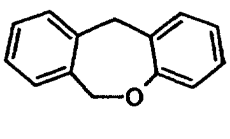, 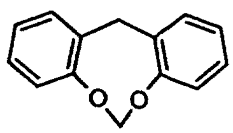,

wobei als Substituenten genannt seien:
Cyano, Nitro, Fluor, Chlor, Brom, Methyl, Ethyl, n- oder i-Propyl, Methoxy, Trifluormethyl oder Dimethylamino oder gegebenenfalls substituiertes Phenyl, wobei als Substituenten für den Phenylrest die Substituenten genannt seien, die unter $R^6$ als Substituenten von Phenyl und Naphthyl genannt sind, und

X für eine Einfachbindung oder für eine der Gruppierungen $-CH_2-$, $-(CH_2)_2-$, $-(CH_2)_3-$, $-CH(CH_3)-$, $-C(CH_3)_2-$, $-CH_2C(CH_3)_2-$, $-CH(CH_3)CH_2-$, $-CH_2O-$, $-CH_2S-$, $-(CH_2)_2O-$, $-(CH_2)_2S-$, $-CH_2OCH_2-$, $CH_2SCH_2-$, $-C(CH_3)=CH$, $-C\equiv C-$, $-(CH_2)_3-N(CH_3)-$, $-(CH_2)_3-N(C_2H_5)-$, $-(CH_2)_3-N(CH_3)-CH_2-$ oder $-(CH_2)_3-N(C_2H_5)-CH_2-$ steht.

**2.** Verbindungen gemäß Anspruch 1, **dadurch gekennzeichnet, dass**

$R^1$ für Wasserstoff, für jeweils gegebenenfalls durch Cyano, Fluor, Chlor, Methoxy oder Ethoxy substituiertes Methyl, Ethyl, n- oder i-Propyl und n-, i-, s- oder t-Butyl; für jeweils gegebenenfalls einfach bis dreifach, gleich oder verschieden durch Fluor, Chlor, Brom, Methyl oder Ethyl substituiertes Cyclopropyl, Cyclopentyl und Cyclohexyl oder für einfach bis dreifach, gleich oder verschieden substituiertes Phenyl steht, wobei als Substituenten genannt seien:
Cyano, Nitro, Amino, Fluor, Chlor, Brom; jeweils gegebenenfalls durch Fluor oder Chlor substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylamino, Ethylamino, n- oder i-Propylamino oder Dimethylamino; jeweils gegebenenfalls durch Fluor und/oder Chlor substituiertes Acetyl, Propionyl, Methoxycarbonyl, Ethoxycarbonyl, n- oder i-Propoxycarbonyl, Methylthio, Methylsulfinyl, Methylsulfonyl, Ethylthio, Ethylsulfinyl oder Ethylsulfonyl; Aminocarbonyl, Aminothiocarbonyl, Methylaminocarbonyl, Ethylaminocarbonyl, n- oder i-Propylaminocarbonyl, Dimethylaminocarbonyl, Diethylaminocarbonyl, Aminosulfonyl, Methylaminosulfonyl, Ethylaminosulfonyl, Dimethylaminosulfonyl und Diethylaminosulfonyl; sowie jeweils gegebenenfalls durch Cyano, Nitro, Fluor, Chlor, Methyl, Ethyl, n- oder i-Propyl, n-, i-, s- oder t-Butyl, Trifluormethyl, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, Difluormethoxy oder Trifluormethoxy, Trifluormethylthio, Trifluormethylsulfinyl oder Trifluormethylsulfonyl substituiertes Phenyl, Phenoxy, Phenylthio, Phenylsulfinyl, Phenylsulfonyl, Benzyl oder Phenylamino,

$R^2$ für Wasserstoff, für jeweils gegebenenfalls durch Cyano, Fluor, Chlor, Methoxy oder Ethoxy substituiertes Methyl, Ethyl, n- oder i-Propyl und n-, i-, s- oder t-Butyl, oder für jeweils gegebenenfalls einfach bis dreifach, gleich oder verschieden durch Fluor, Chlor, Brom, Methyl oder Ethyl substituiertes Cyclopropyl, Cyclopentyl und Cyclohexyl steht, und

$R^4$ und $R^5$ unabhängig voneinander für Wasserstoff, für jeweils gegebenenfalls durch Cyano, Fluor, Chlor, Methoxy oder Ethoxy substituiertes Methyl, Ethyl, n- oder i-Propyl und n-, i-, s- oder t-Butyl, oder für jeweils gegebenenfalls einfach bis dreifach, gleich oder verschieden durch Fluor, Chlor, Brom, Methyl oder Ethyl substituiertes Cyclopropyl, Cyclopentyl oder Cyclohexyl stehen.

**3.** Verbindungen gemäß Anspruch 1 oder 2, **dadurch gekennzeichnet, dass**

$R^2$ für Wasserstoff, Methyl, Ethyl, n- oder i-Propyl oder Cyclopropyl steht.

**4.** Verfahren zur Herstellung der Verbindungen der allgemeinen Formel (Ib) gemäß einem der Ansprüche 1 bis 3, **dadurch gekennzeichnet, daß** man Ethanimidsäureester der Formel (II)

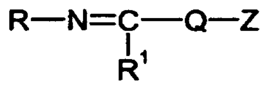

(II)

in welcher

R für CN steht,

$R^1$ die in Anspruch 1 oder 2 angegebene Bedeutung hat,

Q für Sauerstoff oder Schwefel steht und

Z für $C_1$-$C_4$-Alkyl steht,

mit Aminen der Formel (III)

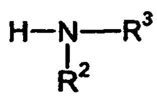

(III)

in welcher

$R^2$ die in einem der Ansprüche 1 bis 3 angegebene Bedeutung hat, und

$R^3$ für

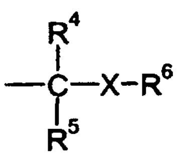

steht, worin $R^4$, $R^5$ und $R^6$ die in Anspruch 1 oder 2 angegebene Bedeutung haben,

in Gegenwart eines Verdünnungsmittels umsetzt.

**5.** Schädlingsbekämpfungsmittel, **gekennzeichnet durch** einen Gehalt an mindestens einer Verbindung der Formel (Ib) gemäß Anspruch 1.

**6.** Verwendung von Verbindungen der Formel (Ib) gemäß Anspruch 1 zur Bekämpfung von Schädlingen.

**7.** Verfahren zur Bekämpfung von Schädlingen, **dadurch gekennzeichnet, daß** man Verbindungen der Formel (Ib) gemäß Anspruch 1 auf Schädlinge und/oder ihren Lebensraum einwirken läßt.

**8.** Verwendung von Imidamid-Derivaten der Formel (I) gemäß Anspruch 1 zur Herstellung von Schädlingsbekämpfungsmitteln.

## Claims

**1.** Compounds of the formula (Ib)

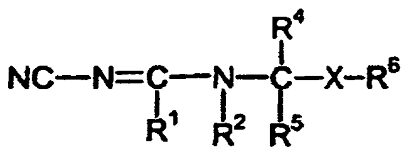

(I b)

in which:

$R^1$ represents hydrogen, represents optionally halogen-, cyano- or $C_1$-$C_4$-alkoxy-substituted $C_1$-$C_4$-alkyl, represents $C_3$-$C_6$-cycloalkyl which is optionally mono- to trisubstituted by identical or different substituents from the group consisting of halogen and $C_1$-$C_4$-alkyl or represents phenyl which is mono- to trisubstituted by identical or different substituents, possible substituents being:
hydroxy, amino, cyano, nitro, halogen; in each case optionally hydroxy-, cyano- or halogen-substituted $C_1$-$C_4$-alkyl, $C_1$-$C_4$-alkoxy, $C_1$-$C_4$-alkylamino and di-($C_1$-$C_4$-)alkylamino; in each case optionally halogen-substituted $C_1$-$C_4$-alkyl-carbonyl, $C_1$-$C_4$-alkoxy-carbonyl, $C_1$-$C_4$-alkyl-carbonyl-amino, $C_1$-$C_4$-alkylthio, $C_1$-$C_4$-alkylsulphinyl and $C_1$-$C_4$-alkylsulphonyl; aminocarbonyl, aminothiocarbonyl, $C_1$-$C_4$-alkylaminocarbonyl, di-($C_1$-$C_4$)-alkylamino-carbonyl, aminosulphonyl, $C_1$-$C_4$-alkylaminosulphonyl and di-($C_1$-$C_4$)-alkylaminosulphonyl; and also phenyl, phenoxy, phenylthio, phenylsulphinyl, phenylsulphonyl, benzyl and phenylamino, each of which is optionally substituted by hydroxy, cyano, nitro, halogen, $C_1$-$C_4$-alkyl, $C_1$-$C_4$-alkoxy, $C_1$-$C_4$-alkylthio, $C_1$-$C_4$-alkylsulphinyl, $C_1$-$C_4$-alkylsulphonyl and $C_1$-$C_4$-haloalkyl, $C_1$-$C_4$-haloalkoxy, $C_1$-$C_4$-haloalkylthio, $C_1$-$C_4$-haloalkylsulphinyl and $C_1$-$C_4$-haloalkylsulphonyl having in each case 1 to 5 identical or different halogen atoms from the group consisting of fluorine, chlorine and bromine,

$R^2$ represents hydrogen, represents optionally halogen-, cyano- or $C_1$-$C_4$-alkoxy-substituted $C_1$-$C_4$-alkyl or represents $C_3$-$C_6$-cycloalkyl which is optionally mono- to trisubstituted by identical or different substituents from the group consisting of halogen and $C_1$-$C_4$-alkyl,

$R^4$ and $R^5$ independently of one another represent hydrogen, represent optionally halogen-, cyano- or $C_1$-$C_4$-alkoxy-substituted $C_1$-$C_4$-alkyl or represent $C_3$-$C_6$-cycloalkyl which is optionally mono- to trisubstituted by identical or different substituents from the group consisting of halogen and $C_1$-$C_4$-alkyl,

$R^6$ represents naphthyl or phenyl which is optionally mono- to trisubstituted by identical or different substituents, possible substituents being:
cyano, nitro, amino, fluorine, chlorine, bromine, methyl, ethyl, n- or i-propyl, n-, i-, s- or t-butyl, methoxy, ethoxy, n- or i-propoxy, trifluoromethyl, difluoromethyl, trifluoromethoxy, difluoromethoxy, trifluoromethylthio, methylamino, dimethylamino, acetyl, propionyl, methoxycarbonyl, ethoxycarbonyl, methylthio, methylsulphinyl, methylsulphonyl, methylaminocarbonyl, ethylaminocarbonyl, n- or i-propylaminocarbonyl, dimethylaminocarbonyl and diethylaminocarbonyl; and also phenyl, phenoxy, phenylthio, phenylsulphinyl, phenylsulphonyl, benzyl or phenylamino, each of which is optionally substituted by cyano, nitro, fluorine, chlorine, methyl, methoxy, methylthio, methylsulphinyl, methylsulphonyl, trifluoromethyl or trifluoromethoxy, trifluoromethylthio, trifluoromethylsulphinyl or trifluoromethylsulphonyl,
represents furthermore cyclopentane or cyclohexane, each of which is optionally mono- to trisubstituted by identical or different substituents from the group consisting of fluorine, chlorine, methyl, ethyl, i-propyl, methoxy, ethoxy, trifluoromethyl or trifluoromethoxy,
and also represents the following heterocycles which are optionally mono- to pentasubstituted by identical or different substituents:

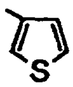, 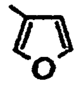, 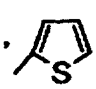, 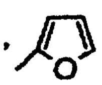, 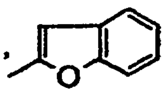, 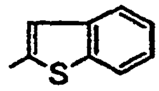, 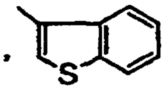,

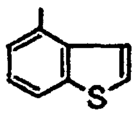, 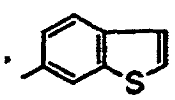, 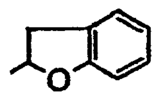, 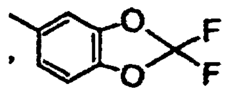, 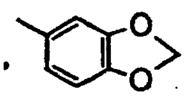,

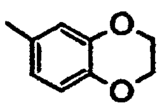, 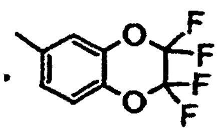, 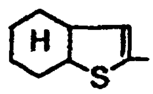, 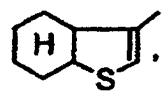, 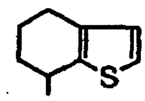;

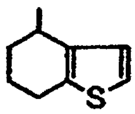, 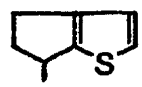, 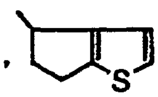, 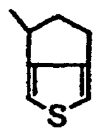, 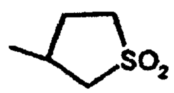, 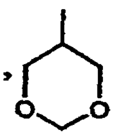,

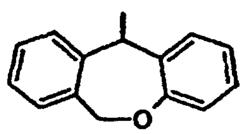, 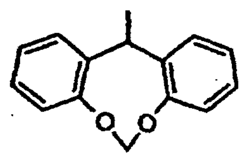,

possible substituents being:
cyano, nitro, fluorine, chlorine, bromine, methyl, ethyl, n- or i-propyl, methoxy, trifluoromethyl or dimethylamino or optionally substituted phenyl, possible substituents for the phenyl radical being the substituents mentioned under $R^6$ as substituents of phenyl and naphthyl, and

X represents a single bond or represents one of the groupings $-CH_2-$, $-(CH_2)_2-$, $-(CH_2)_3-$, $-CH(CH_3)-$, $-C(CH_3)_2-$, $-CH_2C(CH_3)_2-$, $-CH(CH_3)CH_2-$, $-CH_2O-$, $-CH_2S-$, $-(CH_2)_2O-$, $-(CH_2)_2S-$, $-CH_2OCH_2-$, $CH_2SCH_2-$, $-C(CH_3)=CH$, $-C{\equiv}C-$, $-(CH_2)_3-N(CH_3)-$, $-(CH_2)_3-N(C_2H_5)-$, $-(CH_2)_3-N(CH_3)-CH_2$ or $-(CH_2)_3-N(C_2H_5)-CH_2-$.

**2.** Compounds according to Claim 1, **characterized in that**

$R^1$ represents hydrogen, represents in each case optionally cyano-, fluorine- or chlorine-, methoxy- or ethoxy-substituted methyl, ethyl, n- or i-propyl and n-, i-, s- or t-butyl; represents cyclopropyl, cyclopentyl and cyclohexyl, each of which is optionally mono- to trisubstituted by identical or different substituents from the group consisting of fluorine, chlorine, bromine, methyl and ethyl, or represents phenyl which is mono- to trisubstituted by identical or different substituents, possible substituents being:
cyano, nitro, amino, fluorine, chlorine, bromine; in each case optionally fluorine- or chlorine-substituted methyl, ethyl, n- or i-propyl, n-, i-, s- or t-butyl, methoxy, ethoxy, n- or i-propoxy, n-, i-, s- or t-butoxy, methylamino, ethylamino, n- or i-propylamino or dimethylamino; in each case optionally fluorine- and/or chlorine-substituted acetyl, propionyl, methoxycarbonyl, ethoxycarbonyl, n- or i-propoxycarbonyl, methylthio, methylsulphinyl, methylsulphonyl, ethylthio, ethylsulphinyl or ethylsulphonyl; aminocarbonyl, aminothiocarbonyl, methylaminocarbonyl, ethylaminocarbonyl, n- or i-propylaminocarbonyl, dimethylaminocarbonyl, diethylaminocarbonyl, aminosulphonyl, methylaminosulphonyl, ethylaminosulphonyl, dimethylaminosulphonyl and diethylaminosulphonyl; and also phenyl, phenoxy, phenylthio, phenylsulphinyl, phenylsulphonyl, benzyl or phenylamino, each of which

is optionally substituted by cyano, nitro, fluorine, chlorine, methyl, ethyl, n- or i-propyl, n-, i-, s- or t-butyl, trifluoromethyl, methoxy, ethoxy, n- or i-propoxy, methylthio, ethylthio, n- or i-propylthio, methylsulphinyl, ethylsulphinyl, n- or i-propylsulphinyl, methylsulphonyl, ethylsulphonyl, n- or i-propylsulphonyl, difluoromethoxy or trifluoromethoxy, trifluoromethylthio, trifluoromethylsulphinyl or trifluoromethylsulphonyl,

$R^2$ represents hydrogen, or represents in each case optionally cyano-, fluorine- or chlorine-, methoxy- or ethoxy-substituted methyl, ethyl, n- or i-propyl and n-, i-, s- or t-butyl, or represents cyclopropyl, cyclopentyl and cyclohexyl, each of which is optionally mono- to trisubstituted by identical or different substituents from the group consisting of fluorine, chlorine, bromine, methyl and ethyl, and

$R^4$ and $R^5$ independently of one another represent hydrogen, represent in each case optionally cyano-, fluorine-, or chlorine-, methoxy- or ethoxy-substituted methyl, ethyl, n- or i-propyl and n-, i-, s- or t-butyl, or represent cyclopropyl, cyclopentyl or cyclohexyl, each of which is optionally mono- to trisubstituted by identical or different substituents from the group consisting of fluorine, chlorine, bromine, methyl and ethyl.

**3.** Compounds according to Claim 1 or 2, **characterized in that**

$R^2$ represents hydrogen, methyl, ethyl, n- or i-propyl or cyclopropyl.

**4.** Process for preparing compounds of the general formula (Ib) according to any of Claims 1 to 3, **characterized in that** ethaneimidic acid esters of the formula (II)

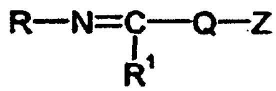
(II)

in which

R represents CN,

$R^1$ has the meaning given in Claim 1 or 2,

Q represents oxygen or sulphur and

Z represents $C_1$-$C_4$-alkyl

are reacted with amines of the formula (III)

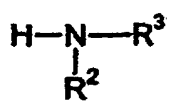
(III)

in which

$R^2$ has the meaning given in any of Claims 1 to 3, and

$R^3$ represents

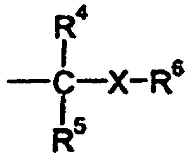

,

where $R^4$, $R^5$ and $R^6$ have the meaning given in Claim 1 or 2,

in the presence of a diluent.

**5.** Pesticides, **characterized in that** they comprise at least one compound of the formula (Ib) according to Claim 1.

**6.** Use of compounds of the formula (Ib) according to Claim 1 for controlling pests.

**7.** Method for controlling pests, **characterized in that** compounds of the formula (Ib) according to Claim 1 are allowed to act on pests and/or their habitat.

**8.** Use of imidamide derivatives of the formula (I) according to Claim 1 for preparing pesticides.

**Revendications**

**1.** Composés de formule (Ib)

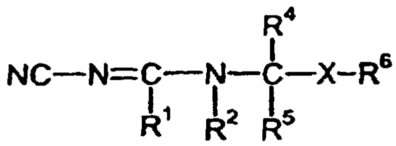

(Ib)

dans laquelle

$R^1$ représente l'hydrogène, un reste alkyle en $C_1$ à $C_4$ éventuellement substitué par un halogène, un radical cyano ou alkoxy en $C_1$ à $C_4$, un reste cycloalkyle en $C_3$ à $C_6$ portant éventuellement un à trois substituants, identiques ou différents, halogéno ou alkyle en $C_1$ à $C_4$, ou un reste phényle portant un à trois des substituants suivants, identiques ou différents :
hydroxy, amino, cyano, nitro, halogéno ; alkyle en $C_1$ à $C_4$, alkoxy en $C_1$ à $C_4$, alkylamino en $C_1$ à $C_4$ et di- (alkyle en $C_1$ à $C_4$)-amino portant chacun le cas échéant un substituant hydroxy, cyano ou halogéno ; (alkyle en $C_1$ à $C_4$)-carbonyle, (alkoxy en $C_1$ à $C_4$)-carbonyle, (alkyle en $C_1$ à $C_4$)-carbonylamino, alkylthio en $C_1$ à $C_4$, alkylsulfinyle en $C_1$ à $C_4$ et alkylsulfonyle en $C_1$ à $C_4$ portant chacun le cas échéant un substituant halogéno ; aminocarbonyle, aminothiocarbonyle, (alkyle en $C_1$ à $C_4$)-aminocarbonyle, di-(alkyle en $C_1$ à $C_4$)-aminocarbonyle, aminosulfonyle, alkylaminosulfonyle en $C_1$ à $C_4$ et di-(alkyle en $C_1$ à $C_4$)-aminosulfonyle ; ainsi que phényle, phénoxy, phénylthio, phénylsulfinyle, phénylsulfonyle, benzyle et phénylamino portant chacun le cas échéant un substituant hydroxy, cyano, nitro, halogéno, alkyle en $C_1$ à $C_4$, alkoxy en $C_1$ à $C_4$, alkylthio en $C_1$ à $C_4$, alkylsulfinyle en $C_1$ à $C_4$, alkylsulfonyle en $C_1$ à $C_4$ et halogénalkyle en $C_1$ à $C_4$, halogénalkoxy en $C_1$ à $C_4$, halogénalkylthio en $C_1$ à $C_4$, halogénalkylsulfinyle en $C_1$ à $C_4$ et halogénalkylsulfonyle en $C_1$ à $C_4$ ayant chacun 1 à 5 atomes d'halogènes, identiques ou différents, de la série fluor, chlore et brome,

$R^2$ représente l'hydrogène, un reste alkyle en $C_1$ à $C_4$ portant éventuellement un substituant halogéno, cyano ou alkoxy en $C_1$ à $C_4$, ou un reste cycloalkyle en $C_3$ à $C_6$ portant éventuellement un à trois substituants, identiques ou différents, halogène ou alkyle en $C_1$ à $C_4$,

$R^4$ et $R^5$ représentent indépendamment l'un de l'autre l'hydrogène, un reste alkyle en $C_1$ à $C_4$ portant éventuellement un substituant halogéno, cyano ou alkoxy en $C_1$ à $C_4$, ou un reste cycloalkyle en $C_3$ à $C_6$ portant éventuellement un à trois substituants, identiques ou différents, halogéno ou alkyle en $C_1$ à $C_4$,

$R^6$ est un reste phényle ou naphtyle portant éventuellement un à trois des substituants suivants, identiques ou différents :

cyano, nitro, amino, fluoro, chloro, bromo, méthyle, éthyle, n-propyle, isopropyle, n-butyle, isobutyle, sec.-butyle, tertiobutyle, méthoxy, éthoxy, n-propoxy, isopropoxy, trifluorométhyle, difluorométhyle, trifluorométhoxy, difluorométhoxy, trifluorométhylthio, méthylamino, diméthylamino, acétyle, propionyle, méthoxycarbonyle, éthoxycarbonyle, méthylthio, méthylsulfinyle, méthylsulfonyle, méthylaminocarbonyle, éthylaminocarbonyle, n-propylaminocarbonyle, isopropylaminocarbonyle, diméthylaminocarbonyle et diéthylaminocarbonyle ; ainsi que phényle, phénoxy, phénylthio, phénylsulfinyle, phénylsulfonyle, benzyle ou phénylamino portant chacun le cas échéant un substituant cyano, nitro, fluoro, chloro, méthyle, méthoxy, méthylthio, méthylsulfinyle, méthylsulfonyle, trifluorométhyle ou trifluorométhoxy, trifluorométhylthio, trifluorométhylsulfinyle ou trifluorométhylsulfonyle,

en outre, cyclopentane ou cyclohexane portant chacun le cas échéant un à trois substituants, identiques ou différents, fluoro, chloro, méthyle, éthyle, isopropyle, méthoxy, éthoxy, trifluorométhyle ou trifluorométhoxy,

de même que les hétérocycles suivants portant éventuellement un à cinq substituants identiques ou différents :

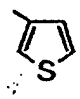, 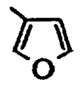, 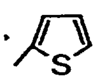, 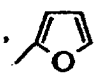, 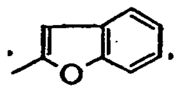, 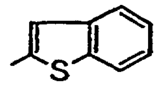, 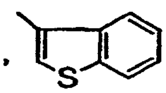,

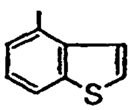, 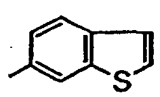, 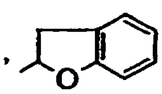, 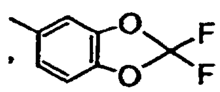, 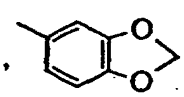,

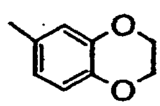, 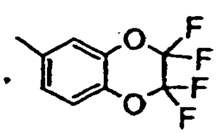, 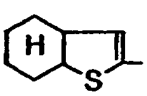, 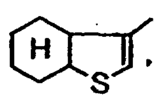, 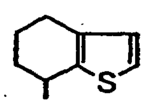;

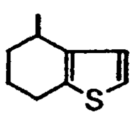, 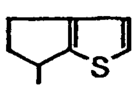, 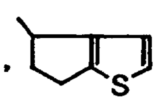, 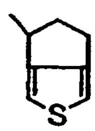, 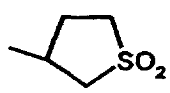, 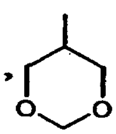,

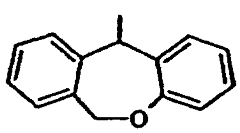, 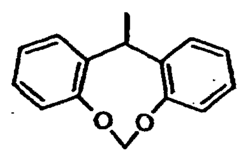.

avec comme substituants :

cyano, nitro, fluoro, chloro, bromo, méthyle, éthyle, n-propyle, isopropyle, méthoxy, trifluorométhyle ou diméthylamino, ou phényle éventuellement substitué, les substituants que l'on peut mentionner pour le reste phényle étant les substituants qui sont mentionnés pour $R^6$ comme substituants des restes phényle et naphtyle, et

X est une liaison simple ou l'un des groupements $-CH_2-$, $-(CH_2)_2-$, $-(CH_2)_3-$, $-CH(CH_3)-$, $-C(CH_3)_2-$, $-CH_2C(CH_3)_2-$, $-CH(CH_3)CH_2-$, $CH_2O-$, $CH_2S-$, $-(CH_2)_2O-$, $-(CH_2)_2S-$, $-CH_2OCH_2-$, $-CH_2SCH_2-$, $-C(CH_3)=CH$, $-C\equiv C-$, $-(CH_2)_3-N(CH_3)-$, $-(CH_2)_3-N(C_2H_5)-$, $-(CH_2)_3-N(CH_3)-CH_2-$ ou $-(CH_2)_3-N(C_2H_5)-CH_2-$.

2. Composés suivant la revendication 1, **caractérisés en ce que**

$R^1$ représente l'hydrogène, un reste méthyle, éthyle, n-propyle, isopropyle, n-butyle, isobutyle, sec.-butyle ou tertiobutyle portant chacun le cas échéant un substituant cyano, fluoro, chloro, méthoxy ou éthoxy ; un reste cyclopropyle, cyclopentyle ou cyclohexyle portant chacun le cas échéant un à trois substituants, identiques ou différents, fluoro, chloro, bromo, méthyle ou éthyle, ou bien un reste phényle portant un à trois substituants identiques ou différents, tels que :
cyano, nitro, amino, fluoro, chloro, bromo ; méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthylamino, éthylamino, n-propylamino, isopropylamino ou diméthylamino portant chacun le cas échéant un substituant fluoro ou chloro ; acétyle, propionyle, méthoxycarbonyle, éthoxycarbonyle, n-propoxycarbonyle, isopropoxycarbonyle, méthylthio, méthylsulfinyle, méthylsulfonyle, éthylthio, éthylsulfinyle ou éthylsulfonyle portant chacun le cas échéant un substituant fluoro et/ou chloro ; aminocarbonyle, aminothiocarbonyle, méthylaminocarbonyle, éthylaminocarbonyle, n-propylaminocarbonyle, isopropylaminocarbonyle, diméthylaminocarbonyle, diéthylaminocarbonyle, aminosulfonyle, méthylaminosulfonyle, éthylaminosulfonyle, diméthylaminosulfonyle et diéthylaminosulfonyle ; ainsi que phényle, phénoxy, phénylthio, phénylsulfinyle, phénylsulfonyle, benzyle ou phénylamino portant chacun le cas échéant un substituant cyano, nitro, fluoro, chloro, méthyle, éthyle, n-propyle, isopropyle, n-butyle, isobutyle, sec.-butyle, tertiobutyle, trifluorométhyle, méthoxy, éthoxy, n-propoxy, isopropoxy, méthylthio, éthylthio, n-propylthio, isopropylthio, méthylsulfinyle, éthylsulfinyle, n-propylsulfinyle, isopropylsulfinyle, méthylsulfonyle, éthylsulfonyle, n-propylsulfonyle, isopropylsulfonyle, difluorométhoxy, trifluorométhoxy, trifluorométhylthio, trifluorométhylsulfinyle ou trifluorométhylsulfonyle,

$R^2$ représente l'hydrogène, un reste méthyle, éthyle, n-propyle, isopropyle, n-butyle, isobutyle, sec.-butyle ou tertiobutyle portant chacun le cas échéant un substituant cyano, fluoro, chloro, méthoxy ou éthoxy, ou bien les restes cyclopropyle, cyclopentyle et cyclohexyle portant chacun le cas échéant un à trois substituants fluoro, chloro, bromo, méthyle ou éthyle identiques ou différents, et

$R^4$ et $R^5$ représentent, indépendamment l'un de l'autre, l'hydrogène, un reste méthyle, éthyle, n-propyle, isopropyle, n-butyle, isobutyle, sec.-butyle ou tertiobutyle portant chacun le cas échéant un substituant cyano, fluoro, chloro, méthoxy ou éthoxy, ou un reste cyclopropyle, cyclopentyle ou cyclohexyle portant chacun le cas échéant un à trois substituants fluoro, chloro, bromo, méthyle ou éthyle identiques ou différents.

**3.** Composés suivant la revendication 1 ou 2, **caractérisés en ce que**

$R^2$ représente l'hydrogène, un reste méthyle, éthyle, n-propyle, isopropyle ou cyclopropyle.

**4.** Procédé de production des composés de formule générale (Ib) suivant l'une des revendications 1 à 3, **caractérisé en ce qu'**on fait réagir des esters d'acides éthanimidiques de formule (II)

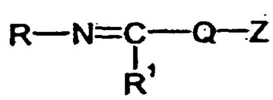 (II)

dans laquelle

R représente CN,
$R^1$ a la définition indiquée dans la revendication 1 ou 2,
Q représente l'oxygène ou le soufre et
Z est un reste alkyle en $C_1$ à $C_4$,

avec des amines de formule (III)

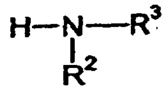 (III)

dans laquelle

$R^2$ a la définition indiquée dans l'une des revendications 1 à 3, et
$R^3$ est un groupe

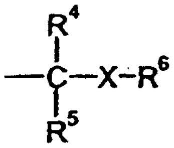

dans lequel $R^4$, $R^5$ et $R^6$ ont la définition indiquée dans la revendication 1 ou 2,

en présence d'un diluant.

**5.** Compositions pesticides, **caractérisées par** une teneur en au moins un composé de formule (Ib) suivant la revendication 1.

**6.** Utilisation de composés de formule (Ib) suivant la revendication 1, pour combattre des parasites.

**7.** Procédé de lutte contre des parasites, **caractérisé en ce qu'**on fait agir des composés de formule (I) suivant la revendication 1 sur les parasites et/ou sur leur milieu.

**8.** Utilisation de dérivés d'imidamides de formule (I) suivant la revendication 1 pour la préparation de compositions pesticides.